# EUROPÄISCHE PATENTSCHRIFT

(11) **EP 3 932 383 B1**
(45) Veröffentlichungstag und Bekanntmachung des Hinweises auf die Patenterteilung: **24.08.2022**
(21) Anmeldenummer: 20184024.6
(22) Anmeldetag: 03.07.2020
(51) Int. Cl.: A61K 6/889, C07C 271/00, C07C 321/12, C07C 323/12, A61K 6/62, A61K 6/818, A61K 6/833

(54) **KOMPOSITE MIT VERRINGERTER POLYMERISATIONSSCHRUMPFUNGSSPANNUNG**
COMPOSITES HAVING REDUCED POLYMERIZATION SHRINKAGE STRESS
COMPOSITE À CONTRAINTE DE RETRAIT DE POLYMÉRISATION RÉDUITE

(43) Veröffentlichungstag der Anmeldung: 05.01.2022
(73) Patentinhaber: Ivoclar Vivadent AG, 9494 Schaan (LI)
(72) Erfinder: MOSZNER, Norbert, 9495 Triesen (LI); CATEL, Yohann, 9475 Sevelen (CH); LAMPARTH, Iris, 9472 Grabs (CH)
(74) Vertreter: Uexküll & Stolberg

(56) Entgegenhaltungen:
- EP-A1- 2 404 916
- EP-A1- 2 965 741
- WO-A1-2006/122081
- US-A1- 2012 295 228

## Beschreibung

Die vorliegende Erfindung betrifft thermisch- und/oder lichthärtende Komposite zur Herstellung von dentalen Zementen und Füllungskompositen, Inlays, Onlays, Kronen, Brücken oder Verblendmaterialien.

Dentale Komposite, die z.B. als Kompositzement oder als direktes Füllungsmaterial, Inlay, Onlay, Krone oder Verblendwerkstoff eingesetzt werden, enthalten eine polymerisierbare organische Matrix und einen oder mehreren Füllstoffe, die meist mit einem polymerisationsfähigen Haftvermittler oberflächenmodifiziert sind. Je nach Art der Füllstoffe, der Monomermatrix und der Anwendung kann der Füllungsgrad zwischen ca. 50 bis 90 Gew.-% variieren, wobei Zemente im Vergleich zu Füllungskompositen einen geringeren Füllungsgrad aufweisen.

Die polymerisierbare organische Matrix enthält in der Regel eine Mischung von Monomeren, Initiator-Komponenten, Stabilisatoren und Pigmenten. Als Harze werden meistens Mischungen von Dimethacrylaten eingesetzt. Beispiele hierfür sind die hochviskosen Dimethacrylate 2,2-Bis[4-(2-hydroxy-3-methacryloyl-oxypropyl)phenyl]propan (Bis-GMA) und 1,6-Bis-[2-methacryloyloxyethoxycarbonylamino]-2,4,4-trimethylhexan (UDMA) oder die als Verdünnermonomere genutzten niedrigviskosen Dimethacrylate, wie z.B. Bismethacryloyloxymethyltricyclo[5.2.1.]-decan (TCDMA), Decandiol-1,10-dimethacrylate (D₃MA) und Triethylenglycoldimethacrylat (TEGDMA).

Bei der radikalischen Polymerisation von dentalen Kompositen kommt es aufgrund des Polymerisationsschrumpfes (ΔV_{P}) der eingesetzten Monomere zu einer Volumenkontraktion, was bei Füllungskompositen zu einer sehr nachteiligen Randspaltbildung führen kann. Bei der Polymerisation von monofunktionellen Methacrylaten, wie z.B. MMA (ΔV_{P} = 21,0 Vol.-%), führt diese Polymerisationsschrumpfung nicht zum Aufbau einer Polymerisationsschrumpfungsspannung (PKS), weil die Verringerung des Volumens durch Fließen der gebildeten Makromoleküle kompensiert werden kann. Im Falle der vernetzenden Polymerisation von multifunktionellen Methacrylaten bildet sich aber schon innerhalb weniger Sekunden am sogenannten Gelpunkt, d.h. schon bei geringem Monomerumsatz, ein dreidimensionales Polymernetzwerk aus, so dass der Polymerisationsschrumpf nicht mehr durch viskoses Fließen kompensiert werden kann und sich im Material mit zunehmendem Monomerumsatz eine erhebliche PKS aufbaut. Dabei ist die Entwicklung der PKS bei Füllungskompositen von zahlreichen Faktoren abhängig, u.a. von der Höhe der Volumenkontraktion während der Polymerisation (Härtung bzw. Nachhärtung), den viskoelastischen Eigenschaften (Elastizitätsmodul und Modulaufbau, Glasübergangstemperatur (T_{G}) von Monomer und Polymer, Viskosität und Fließverhalten), der Polymerisationskinetik der Polymernetzwerkbildung (Harzfunktionalität, Vernetzungsdichte, Anteil an cyclischen Strukturen, Polymerisationsgeschwindigkeit, Temperatur, Monomer- und Doppelbindungsumsatz), der Art der Härtung und der Art der Restauration (Schichtdicke, Kavitätengeometrie). Eine besonders hohe PKS wird bei der Lichthärtung beobachtet (vgl. R. R. Braga, R. Y. Ballester, J. L. Ferracane, Dent. Mater. 21 (2005) 962-970; J. W. Stansbury, Dent. Mater. 28 (2012) 13-22).

Es wurden zahlreiche Strategien zur Reduktion der PKS verfolgt. Das betrifft klinische Methoden, wie z.B. die Inkrement-Schicht-Technik, die Verwendung von Kavitätenlacken mit niedrigem E-Modul zur Bildung einer stressabsorbierden Schicht, der Einsatz von speziellen Belichtungsstrategien (soft-start) oder das Vorerwärmen der Komposite zur Verbesserung der Fließeigenschaften. Der Einsatz von neuen schrumpfungsarmen Monomeren, z.B. von Monomeren mit ringöffnend polymerisierbaren Gruppen, oder die Verwendung von maßgeschneiderten Vernetzern, z.B. mit photo- oder thermolabilen Spacern, kann ebenfalls zu Kompositen mit geringer PKS führen.

Außerdem wurde versucht, die PKS durch den Zusatz von hyperverzweigten Monomeren, Nanogelen oder Nanotubes sowie von Low-Profile-Additiven oder expandierbaren Füllstoffen zu verringern.

Die WO 98/37104 offenbart ein Verfahren zur Kontrolle des Molekulargewichts bei der Herstellung linearer Polymere durch photoinitiierte radikalische Polymerisation von Vinylmonomeren, bei dem der Photoinitiator zusammen mit einem Additions-Fragmentierungs-Kettenübertragungsreagenz eingesetzt wird. Gemäß WO 2006/086646 A2 soll sich die PKS in vernetzten Polymeren durch den Einbau von Gruppen in das Polymernetzwerk verringern lassen, die eine reversible Kettenspaltung ermöglichen. Nach der Härtung werden diese Gruppen beispielsweise durch Bestrahlen mit Licht aktiviert. Dies soll eine reversible Spaltung der Polymerketten bewirken, durch die die PKS abgebaut wird. Zum Einbau dieser Gruppen in die Polymerketten werden reversible Additions-Fragmentierungs-Kettenübertragungs- (RAFT; Reversible Addition-Fragmentation Chain Transfer) Mittel wie z.B. Allylsulfide, Dithiocarbamate und Thiocarbonate eingesetzt. Solche RAFT-Reagenzien sind aus der kontrollierten radikalischen Polymerisation bekannt (vgl. z.B. Moad, G.; Rizzardo, E.; Thang, S. H. Polymer 2008, 49, 1079-1131).

Die US 2012/0295228 A1 offenbart radikalisch polymerisierbare Dentalmaterialien, die ethylenisch ungesättigte Monomere mit Allyldisulfidgruppen enthalten, die als Additions-Fragmentierungs-Materialien wirksam sind und die PKS reduzieren sollen. Die WO 2006/122081 A1, EP 2404916 A1 und EP 2965741 A1 offenbaren weitere polymerisierbare Dentalmaterialien.

Außerdem ist bekannt, dass Mercaptane, wie z.B. Mercaptobenzol, Benzylmercaptan, Mercaptopropionsäuremethylester, 1,6-Hexandithiol oder Trimethylolpropantris-(3-mercaptopropionat), zu einer deutlichen Verringerung der Polymerkettenlänge und zur Bildung homogenerer Netzwerke mit geringeren T_{G}-Werten und engerer Verteilung führen (Hacioglu, B.; Berchtold, K. A.; Lovell, L. G.; Nie, J.; Bowman, C. N. Biomaterials 2002, 23, 4057-4067; Dean, K. M.; Cook, W. D.; Lin, M. Y. Eur. Polym. J. 2006, 42, 2872-2887).

Die US 5,932,675 offenbart ein Verfahren zur Herstellung von Polymeren mit geringem Molekulargewicht durch radikalische Polymerisation. Die Kontrolle des Molekulargewichts erfolgt durch die Zugabe von Kettenübertragungsreagenzien wie z.B. α-(t-Butanthiomethyl) styrol.

Nachteilig an den bekannten Verfahren ist, dass der Zusatz von übertragungsaktiven Verbindungen meist zu einer deutlichen Verringerung der Polymerisationsgeschwindigkeit führt, vor allem im Falle von Schwefelverbindungen. Außerdem ist der Einsatz von Mercaptanen aufgrund Ihres Geruches und der von vielen anderen RAFT-Reagenzien aufgrund ihrer Farbe für dentale Anwendungen praktisch ausgeschlossen.

Der Erfindung liegt die Aufgabe zugrunde, polymerisierbare Dentalwerkstoffe bereitzustellen, die die oben genannten Nachteile nicht aufweisen und die sich im Vergleich zum Stand der Technik durch eine verringerte Polymerisationsschrumpfungsspannung (PKS) bei vergleichbaren mechanischen Eigenschaften und vergleichbarer Polymerisationsgeschwindigkeit auszeichnen. Außerdem sollen die Materialien eine homogenere Netzwerkarchitektur und einen engeren Bereich für die Glasübergangstemperatur aufweisen. Sie sollen darüber hinaus einen für die intraorale Anwendung akzeptablen Geruch und keine Eigenfarbe haben.

Die Aufgabe wird erfindungsgemäß durch Zusammensetzungen gelöst, die mindestens eine Verbindung der Formeln I bis III enthalten:

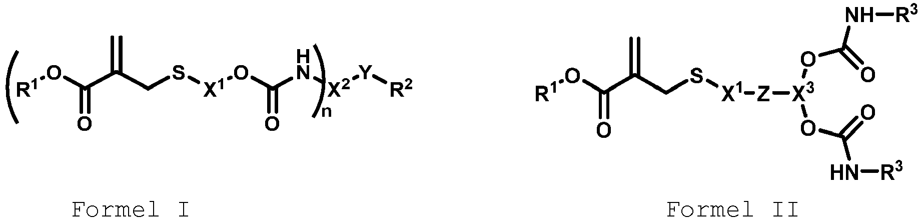

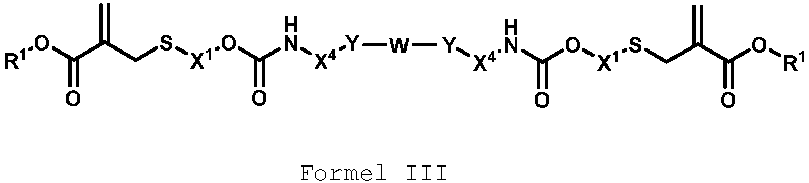

Darin bedeuten:
- R¹: Wasserstoff, ein aliphatischer linearer oder verzweigter C₁-C₁₅-Alkyl-Rest, der durch ein oder mehrere, vorzugsweise 1 bis 3, O oder S unterbrochen und der substituiert oder unsubstituiert sein kann, Benzyl oder Phenyl;
- R²: Wasserstoff, ein aliphatischer linearer oder verzweigter C₁-C₁₅-Alkyl-Rest, der durch ein oder mehrere, vorzugsweise 1 bis 3, O oder S unterbrochen und der substituiert oder unsubstituiert sein kann, Benzyl, ein aromatischer C₆-C₁₈-Rest, der substituiert oder unsubstituiert sein kann, oder eine (Meth)acryloyloxy-Gruppe;
- R³: ein aliphatischer linearer oder verzweigter C₁-C₁₅-Alkyl-Rest, der durch ein oder mehrere, vorzugsweise 1 bis 3, O unterbrochen und der substituiert oder unsubstituiert sein kann, Benzyl, oder ein aromatischer C₆-C₁₈-Rest, der substituiert oder unsubstituiert sein kann;
- X¹: ein linearer oder verzweigter aliphatischer C₁-C₁₅-Alkylen-Rest, der durch ein oder mehrere, vorzugsweise 1 bis 3, O oder S unterbrochen sein kann, oder ein cycloaliphatischer C₆-C₁₆-Rest;
- X²: ein (n+1)-wertiger organischer C₁-C₂₀-Rest, vorzugsweise ein aliphatischer C₁-C₂₀-Rest, der durch ein oder mehrere, vorzugsweise 1 bis 3, O oder S unterbrochen sein kann, ein cycloaliphatischer C₅-C₂₀-Rest, ein aromatischer C₆-C₂₀-Rest oder eine Isocyanursäurerest gemäß der folgenden Formel:

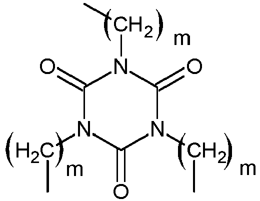

- X³: ein 3-wertiger linearer oder verzweigter aliphatischer C₃-C₂₀-Rest;
- X⁴: ein 2-wertiger organischer C₂-C₃₀-Rest, vorzugsweise ein aliphatischer C₂-C₂₀-Rest, ein cycloaliphatischer C₅-C₂₀-Rest, ein aromatischer C₆-C₂₀-Rest oder Kombinationen davon, wobei diese Reste jeweils durch eine oder mehrere, vorzugsweise 1 bis 3, Methylgruppen substituiert sein können oder unsubstituiert sind;
- Y: entfällt oder eine Ether- (-O-), Ester- (-COO- oder -OOC-), Urethan- (-NR⁴-CO-O- oder -O-CO-NR⁴-) oder Amid-Gruppe (-CONR⁴- oder -NR⁴-CO-), wobei R⁴ jeweils für H oder einen C₁-C₃-Alkyl-Rest steht;
- Z: entfällt oder eine Ether- (-O-), Ester- (-COO- oder -OOC-), oder Amid-Gruppe (-CONR⁵- oder -NR⁵-CO-), wobei R⁵ für H oder einen C₁-C₃-Alkyl-Rest steht; W eine oligomere Polyester-, Polyether- oder Polythioethergruppe;
- m: 1, 2, 3, 4, 5 oder vorzugsweise 6;
- n: 1, 2, 3 oder 4,
wobei dann, wenn X² ein Isocyanursäurerest ist, Y und R² entfallen und n gleich 3 ist.

W hat vorzugsweise eine Molmasse von 500-3000 g/mol. Die Molmasse von W wird anhand der bei der Synthese eingesetzten oligomeren Bausteine bestimmt, z.B. des OH-telechelen Polyethers oder Polyesters. Beispielsweise enthält käufliches OHtelecheles PEG-1000 ca. 22 Wiederholungseinheiten (HO-(CH₂-CH₂-O-)₂₂H mit einer Molmasse von jeweils 44 g/mol. Zusammen mit der endständigen OH-Gruppe und dem endständigen H-Atom ergibt sich ein Molgewicht von 985 g/mol. Die Molmasse der für die Synthese eingesetzten Oligomeren wird vorzugsweise mittels Dampfdruckosmometrie, Ebullioskopie oder Kryoskopie bestimmt. Es handelt sich um die zahlenmittlere, absolute Molmasse.

Bevorzugte Polyethergruppen sind Oligomere aus Ethylen- oder Propylenglycolbausteinen oder Ringöffnungshomo- oder Copolymerisate aus Ethylenoxid, Propylenoxid und/oder Tetrahydrofuran. Bevorzugte Polyestergruppen sind Polykondensate aus aliphatischen C₂-C₁₀-Diolen und C₃-C₁₀-Dicarbonsäuren oder C₃-C₈-α,ω-Hydroxycarbonsäuren sowie Ringöffnungspolymerisate von Caprolacton. Bevorzugte Polythioethergruppen sind Polykondensate von α,ω-Dihalogen-C₂-C₁₀-alkanen mit Natriumsulfid mit maximal fünf Schwefelatomen im Baustein, wobei die erfindungsgemäßen Verbindungen der Formeln I bis III in allen Fällen vorzugsweise jeweils nicht mehr als fünf Schwefelatome pro Molekül enthalten.

Bevorzugte Halogene sind Chlor oder Brom. Die bei den einzelnen Resten ggf. vorhandenen Substituenten sind vorzugsweise jeweils aus C₁-C₃-Alkylgruppen, insbesondere CH₃- und/oder C₂H₅-, Halogen, -OH, -OCH₃ oder -O-COCH₃, polymerisationsfähigen Vinyl-, (Meth)acryloyloxy- und/oder (Meth)acrylamidgruppen ausgewählt. Die Reste können jeweils mit einem oder mit mehreren Substituenten substituiert sein. Vorzugsweise sind die Reste mit 1 bis 3 Substituenten substituiert oder unsubstituiert, wobei insbesondere aliphatische Reste vorzugsweise nicht substituiert sind.

Durch die Formeln I, II und III und allen übrigen hierin gezeigten Formeln werden sämtliche stereoisomere Formen sowie Gemische verschiedener stereoisomerer Formen, wie z.B. Racemate, erfasst. Die Formeln erstrecken sich nur auf solche Verbindungen, die mit der chemischen Valenzlehre vereinbar sind. Der Hinweis, dass ein Rest z.B. durch ein oder mehrere O- oder S-Atome unterbrochen ist, ist so zu verstehen, dass diese Gruppen in die Kohlenstoffkette des Restes eingeschoben werden. Diese Gruppen sind damit beidseitig durch C-Atome begrenzt und können nicht endständig sein. C₁-Reste können beispielsweise nicht unterbrochen, verzweigt, cyclisch oder aromatisch sein. Unter aromatischen Kohlenwasserstoffresten werden der üblichen Nomenklatur entsprechend auch solche Reste verstanden, die aromatische und nicht aromatische Gruppen enthalten.

Die Verbindungen der Formeln I bis III zeichnen sich dadurch aus, dass sie mindestens eine Allylsulfidgruppe der Formel R¹-O-CO-C(=CH₂)-CH₂-S-X¹- und mindestens eine Urethangruppe enthalten, wobei die Anzahl der Urethangruppen vorzugsweise der Anzahl der Allylsulfidgruppen entspricht oder größer ist. Vorzugsweise beträgt das Verhältnis von Allylsulfidgruppen zu Urethangruppen 1:1 bis 1:2. Verbindungen der Formeln I bis III werden hierin auch als Urethanallylsulfide oder Allylsulfide bezeichnet.

Vorzugsweise haben die Variablen die folgenden Bedeutungen:
- R¹: Wasserstoff, ein aliphatischer linearer oder verzweigter C₁-C₁₀-Alkyl-Rest, der durch ein oder mehrere, vorzugsweise 1 bis 2, O unterbrochen sein kann und der einen oder mehrere, vorzugsweise 1 bis 3, Substituenten tragen kann, die vorzugsweise aus -CH₃, -C₂H₅ und/oder polymerisationsfähigen (Meth)acryloyloxygruppen ausgewählt sind, oder unsubstituiert ist, Benzyl oder Phenyl;
- R²: Wasserstoff, ein aliphatischer linearer oder verzweigter C₁-C₁₀-Alkyl-Rest, der durch ein oder mehrere, vorzugsweise 1 bis 2, O unterbrochen sein kann und der ein oder mehrere, vorzugsweise 1 bis 3, Substituenten tragen kann, die vorzugsweise aus -CH₃, -C₂H₅ und/oder polymerisationsfähigen (Meth)acryloyloxygruppen ausgewählt sind, oder unsubstituiert ist, Benzyl, oder ein aromatischer C₆-C₁₂-Rest, der ein oder mehrere, vorzugsweise 1 bis 3, Substituenten tragen kann, die vorzugsweise aus -CH₃, -C₂H₅, und/oder eine polymerisationsfähigen (Meth)acryloyloxygruppen ausgewählt sind, oder unsubstituiert ist, oder eine (Meth)acryloyloxy-Gruppe;
- R³: ein aliphatischer linearer oder verzweigter C₁-C₁₀-Alkyl-Rest, der ein oder mehrere, vorzugsweise 1 bis 3, Substituenten tragen kann, die vorzugsweise aus -CH₃, -C₂H₅, Halogen und/oder polymerisationsfähigen (Meth)acryloyloxygruppen ausgewählt sind, oder unsubstituiert ist, Benzyl, oder ein aromatischer C₆-C₁₈-Rest, der ein oder mehrere, vorzugsweise 1 bis 3, Substituenten tragen kann, die vorzugsweise aus -CH₃, -C₂H₅, polymerisationsfähigen Vinyl- und/oder (Meth)acryloyloxygruppen ausgewählt sind, oder unsubstituiert ist;
- X¹: ein linearer oder verzweigter aliphatischer C₁-C₁₀ Alkylen-Rest, der durch ein oder mehrere, vorzugsweise 1 bis 2, O unterbrochen sein kann, oder ein cycloaliphatischer C₆-C₁₂-Rest;
- X²: ein (n+1)-wertiger organischer C₁-C₁₅-Rest, vorzugsweise ein aliphatischer C₁-C₁₅-Rest, der durch ein oder mehrere, vorzugsweise 1 bis 2, O unterbrochen sein kann, ein cycloaliphatischer C₅-C₁₀-Rest oder ein aromatischer C₆-C₁₄-Rest;
- X³: ein 3-wertiger linearer oder verzweigter aliphatischer C₃-C₁₀-Rest;
- X⁴: ein 2-wertiger organischer C₂-C₂₀-Rest, vorzugsweise ein aliphatischer C₂-C₂₀-Rest Rest, ein cycloaliphatischer C₅-C₁₀-Rest Rest, der eine oder mehrere, vorzugsweise 1 bis 2, Methylgruppen als Substituenten tragen kann oder unsubstituiert ist, oder eine Kombination davon;
- Y: entfällt, oder eine Ester- oder Urethan-Gruppe;
- Z: entfällt oder eine Ether- oder Ester-Gruppe; W eine oligomere Polyester- oder Polyethergruppe und
- n: 1, 2 oder 3.

Besonders bevorzugt haben die Variablen die folgenden Bedeutungen:
- R¹: ein aliphatischer linearer oder verzweigter C₁-C₄-AlkylRest oder Benzyl (Ph-CH₂-), ganz besonders bevorzugt Methyl oder Ethyl;
- R²: Wasserstoff oder eine (Meth)acryloyloxygruppe;
- R³: ein linearer C₁-C₃-Alkyl-Rest, Benzyl (Ph-CH₂-), Phenyl (Ph-) oder p-Tolyl (H₃C-Ph-), wobei diese Reste jeweils durch eine (Meth)acryloyloxygruppe substituiert sein können;
- X¹: ein linearer C₁-C₃ Alkylen-Rest;
- X²: ein (n+1)-wertiger, linearer oder verzweigter, aliphatischer C₂-C₁₂-Rest, der durch 1 bis 3 O unterbrochen sein kann, oder ein aromatischer C₆-C₁₂-Rest;
- X³: ein 3-wertiger, linearer oder verzweigter, aliphatischer C₃-C₄-Rest; insbesondere -CH₂-CH(-)-CH₂-;
- X⁴: ein 2-wertiger aliphatischer C₂-C₁₀-Rest oder ein cycloaliphatischer C₆-Rest, der mit 1 bis 3 Methylgruppen subsituiert sein kann;
- Y: entfällt, eine Ester- oder Urethan-Gruppe;
- Z: entfällt; W eine oligomere Polyester- oder Polyethergruppe und
- n: 1, 2 oder 3, insbesondere 1 oder 2.

Verbindungen der Formel I sind ganz besonders bevorzugt, und am meisten bevorzugt sind Verbindungen der Formel I, in denen die Variablen die folgenden Bedeutungen haben:
- R¹: ein linearer oder verzweigter C₁-C₃-Alkyl-Rest oder Benzyl (Ph-CH₂-), ganz besonders bevorzugt Methyl oder Ethyl;
- R²: Wasserstoff oder eine (Meth)acryloyloxygruppe;
- X¹: ein linearer C₁-C₃ Alkylen-Rest;
- X²: ein (n+1)-wertiger, linearer, aliphatischer C₂-C₁₂-Rest, der durch 1 bis 3 Methylgruppen substituiert sein kann und der durch 1 bis 3 O unterbrochen sein kann, oder ein aromatischer C₆-C₁₂-Rest, der durch 1 bis 4 Methylgruppen substituiert sein kann, wobei der aromatische C₆-C₁₂-Rest bevorzugt aus -Ph-, -CH₂-Ph-, -CH₂-Ph-CH₂- und -C(CH₃)₂-Ph-C(CH₃)₂- ausgewählt ist;
- Y: entfällt oder -O-;
- n: 1 oder 2.

Polymerisationsübertragungsaktive Verbindungen der Formeln I bis III sind nicht bekannt und lassen sich nach bekannten Synthesemethoden herstellen. Beispielsweise können Verbindungen der Formel I in zwei Stufen erhalten werden. Im ersten Schritt entsteht durch Veretherung eines α-Chlormethylacrylats mit einem α,ω-Hydroxyalkylmercaptan ein OH-funktionalisiertes Allylsulfid, das dann in einer zweiten Stufe mit einem geeigneten Isocyanat zu dem erfindungsgemäßen Urethanallylsulfid der Formel I umgesetzt wird:
1. Stufe: 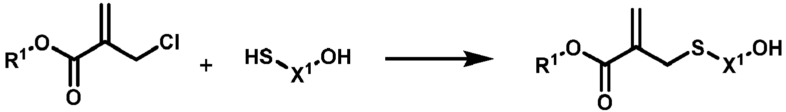 Ein konkretes Beispiel ist: 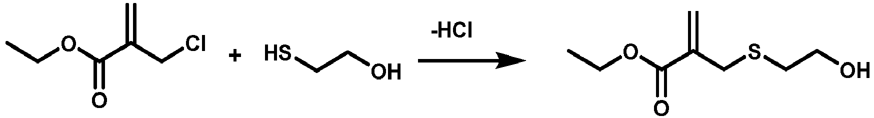
2. Stufe: 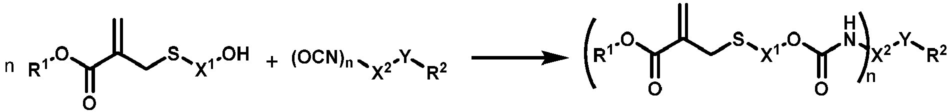 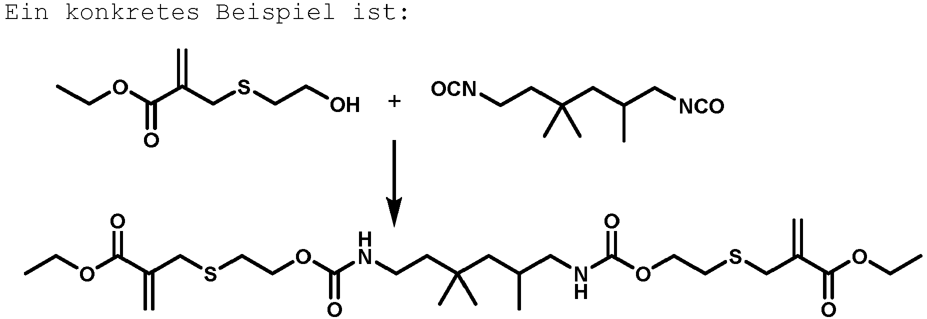
Analog lassen sich die erfindungsgemäßen Urethanallylsulfide der Formel II in zwei Stufen herstellen:
1. Stufe: 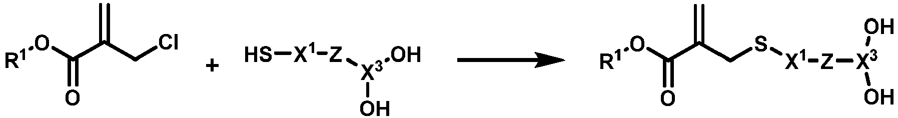 Ein konkretes Beispiel ist: 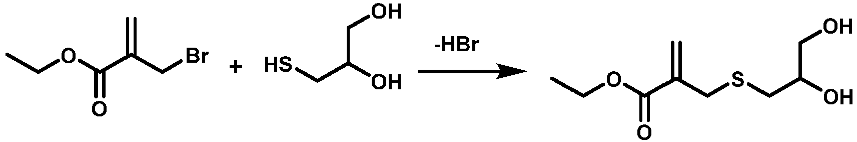
2. Stufe: 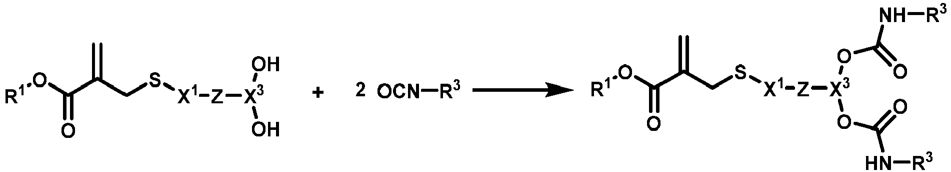 Ein konkretes Beispiel ist:

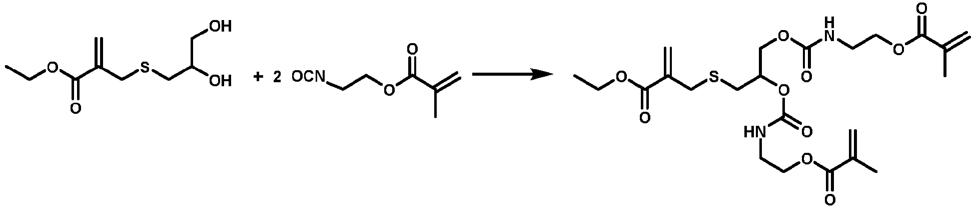

Die erfindungsgemäßen oligomeren Urethanallylsulfide der Formel III lassen sich ebenfalls in zwei Stufen herstellen (z.B. mit Y = Urethan):
1. Stufe: 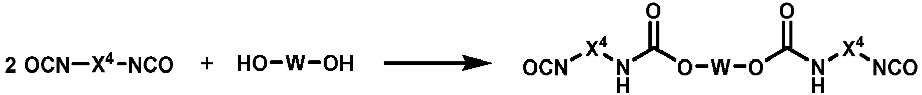 Konkretes Beispiel: 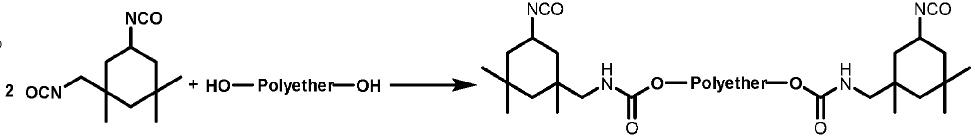
2. Stufe: 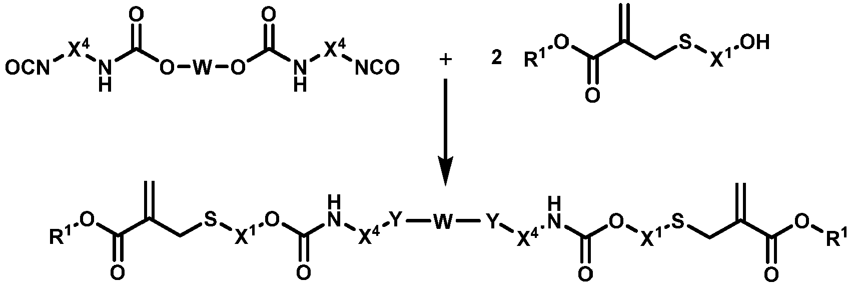 Konkretes Beispiel:

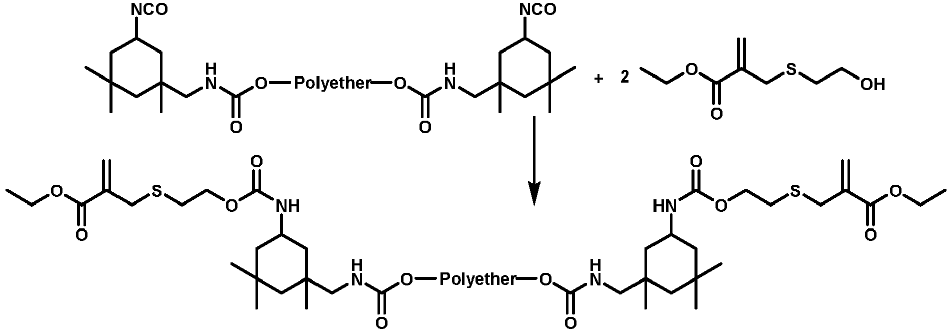

Bevorzugte erfindungsgemäße Urethanallylverbindungen der Formel I sind:

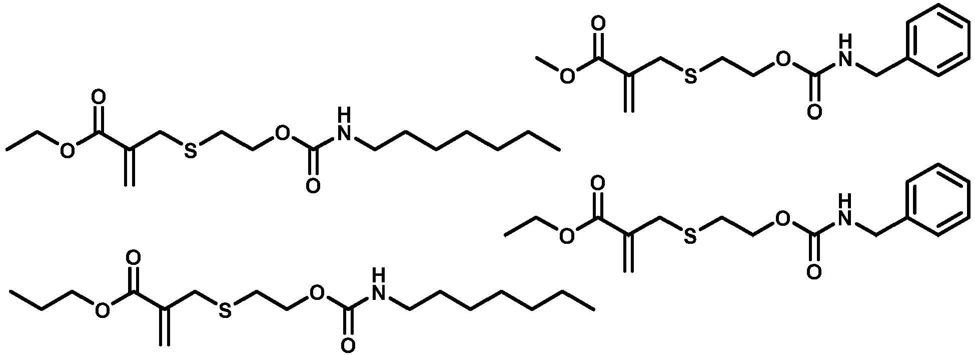

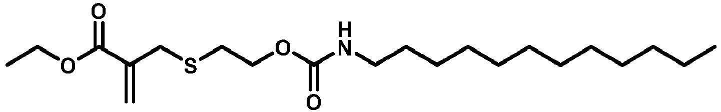

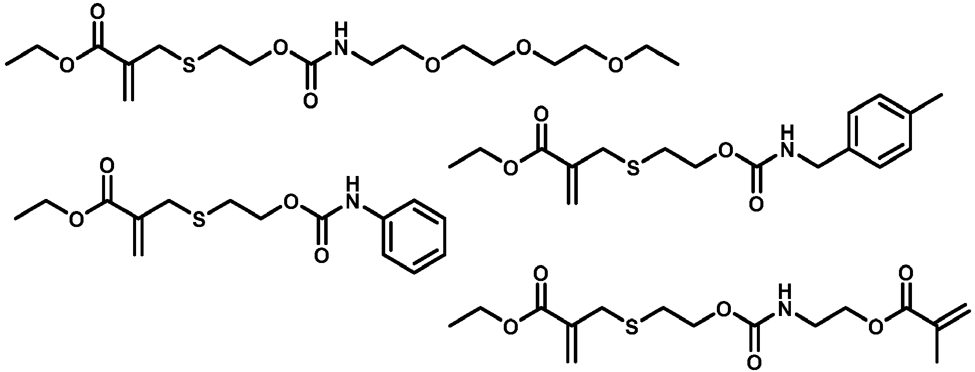

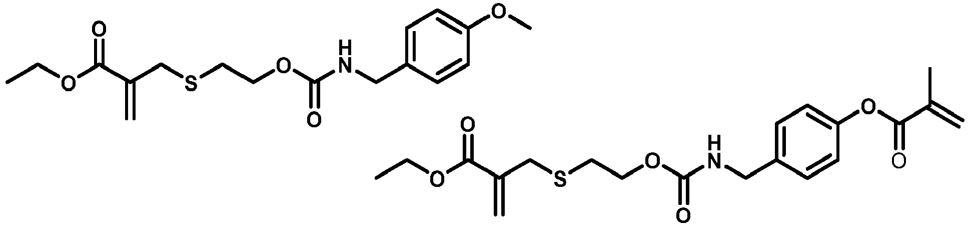

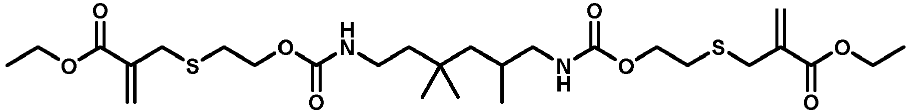

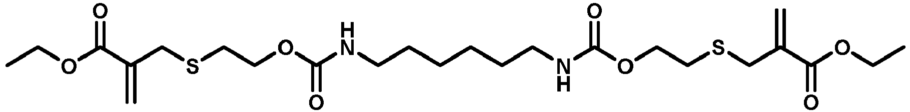

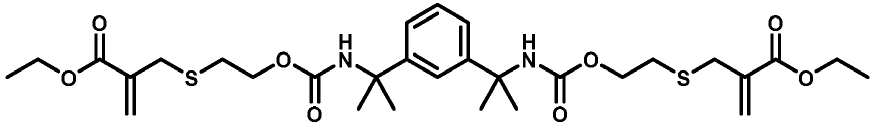

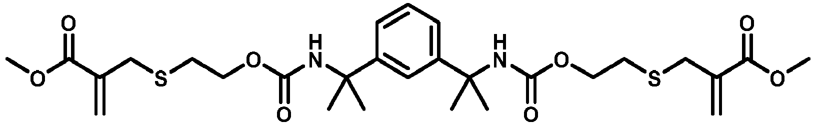

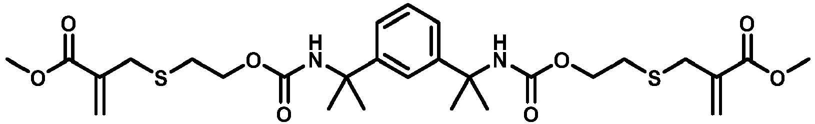

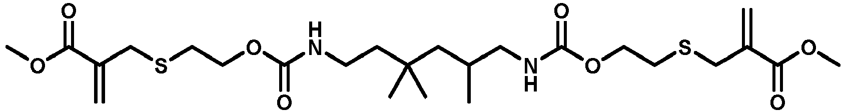

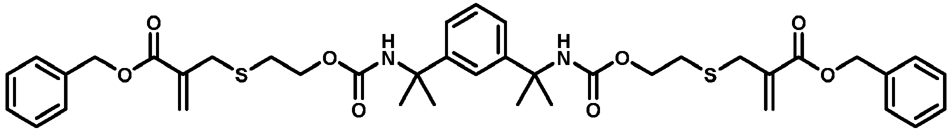

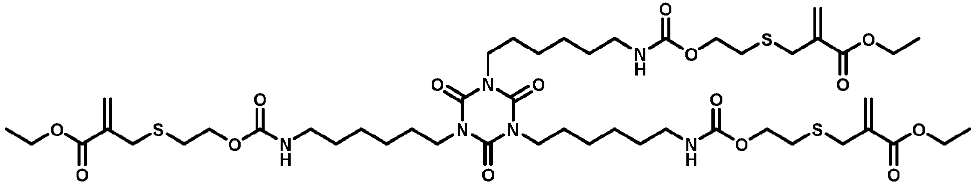

Bevorzugte Urethanallylverbindungen der Formel II sind:

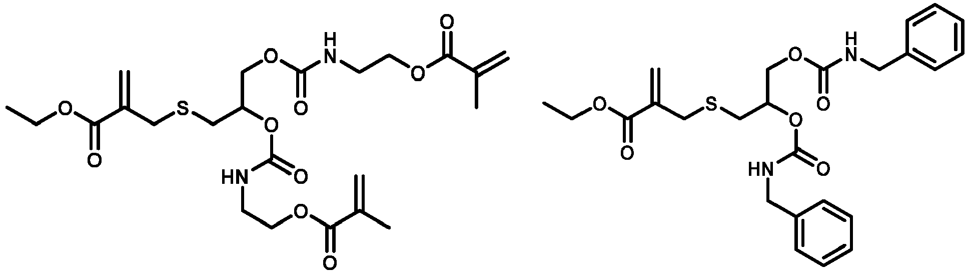

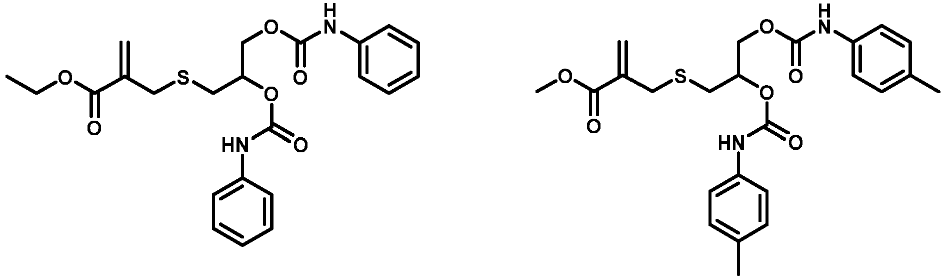

Bevorzugter Urethanallylverbindungen der Formel III sind:

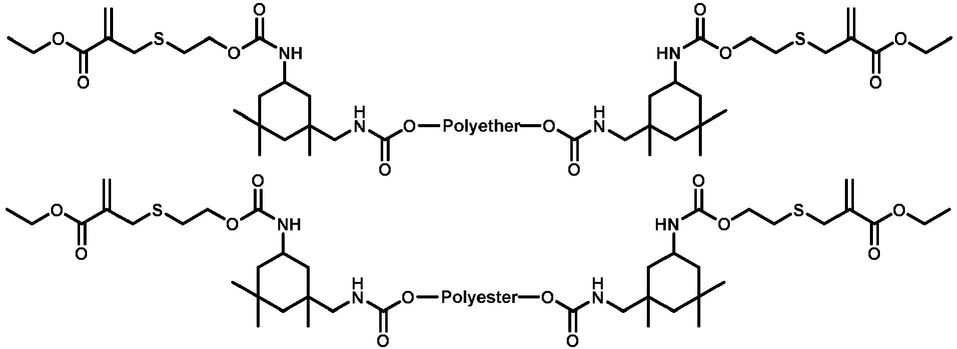

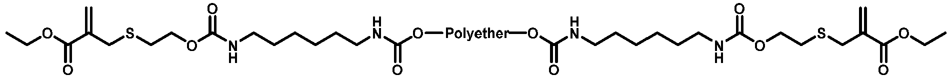

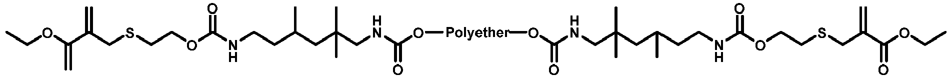

Die Verbindungen der Formeln I bis III ermöglichen eine Kontrolle bzw. Steuerung der Netzwerkstruktur bei der radikalischen Polymerisation. Sie ergeben überraschenderweise homogenere Polymernetzwerke mit einem engeren Glasübergang, d.h., dass der Glasübergang in einem engeren Temperaturbereich stattfindet. Dementsprechend wird auch die PKS während der Aushärtung der Materialien verringert, was für eine dentale Anwendung z.B. als Füllungsmaterial ein großer Vorteil ist.

Die erfindungsgemäßen Zusammensetzungen enthalten neben mindestens einer Verbindung der Formel I, II und/oder III vorzugsweise mindestens ein weiteres radikalisch polymerisierbares Monomer, besonders bevorzugt mindestens ein multifunktionelles (Meth)acrylat oder eine Mischung von mono- und multifunktionellen (Meth)acrylaten. Unter monofunktionellen (Meth)-acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten.

Beispiele für besonders geeignete mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-G(M)A (ein Additionsprodukt aus (Meth)-acrylsäure und Bisphenol-A-diglycidylether), ethoxy- oder propoxyliertes Bisphenol-A-di(meth)acrylat, wie z.B. Bisphenol-A-dimethacrylat mit 3 Ethoxygruppen (SR-348c, Fa. Sartomer) oder 2,2-Bis[4-(2-(meth)acryloxypropoxy)phenyl]propan, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Glycerindi- und tri-(meth)acrylat, 1,4-Butandioldi(meth)acrylat, 1,10-Decandioldi(meth)acrylat (D₃MA) oder 1,12-Dodecandioldi(meth)acrylat.

Geeignet sind außerdem auch thermo- oder photolabile Di(meth)-acrylate, wie z.B. das Additionsprodukt aus 2 mol 2-Acetoacetoxyethylmethacrylat und 1 mol 2,2,4-Trimethylhexamethylen-1,6-diisocyanat (thermolabil) oder Methacrylsäure-2-[2-(4-{2-methyl-2-[2-(methacryloyloxy)-ethylcarbamoyloxy]propionyl}-phenoxy)-ethoxycarbonylamino]-ethylester. Diese eignen sich besonders zur Herstellung von Werkstoffen mit Debonding-on-Demand-Eigenschaften.

Die erfindungsgemäßen dentalen Werkstoffe können neben den oben genannten Monomeren vorzugweise auch radikalisch polymerisierbare, säuregruppenhaltige Monomere (Haftmonomere) enthalten. Bevorzugte Säuregruppen sind Carbonsäuregruppen, Phosphonsäuregruppen und Phosphorsäuregruppen. Bevorzugte säuregruppenhaltige Monomere sind 4-(Meth)acryloyloxyethyl-trimellitsäure¬ianhydrid, 10-Methacryloyloxydecylmalonsäure, N-(2-Hydroxy-3-methacryloyloxypro¬pyl)-N-phenylglycin und 4-Vinylbenzoesäure. Weitere bevorzugte säuregruppenhaltige Monomere sind 2-Methacryloyloxyethyl-phenyl-hydrogen¬phosphat, 10-Methacryloyloxydecyldihydro-genphosphat (MDP) oder Dipentaerythrit¬pentamethacryloyloxyphosphat. Bevorzugt sind außerdem 4-Vinylbenzylphosphonsäure, 2-[4-(Dihydroxyphosphoryl)-2-oxa-bu¬tyl]-acrylsäure. Weiter bevorzugt sind Amide und Ester der genannten säuregruppenhaltigen Monomere, wie z.B. 2-[4-(Di¬hydroxyphosphoryl)-2-oxa-butyl]-acrylsäure-2,4,6-trimethylphenylester sowie (Meth)¬acrylamiddihydrogenphosphate, wie z.B. 6-Methacrylamidohexyl- oder 1,3-Bis¬(methacrylamido)-propan-2-yl-dihydrogenphosphat. Haftmonomere eignen sich besonders zur Herstellung von selbsthaftenden Befestigungszementen.

Zur Initiierung der radikalischen Polymerisation enthalten die erfindungsgemäßen Zusammensetzungen vorzugsweise einen Initiator für die radikalische Polymerisation, besonders bevorzugt einen Photoinitiator. Als Photoinitiatoren eignen sich insbesondere Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil. Bevorzugt werden Campherchinon (CQ) und 2,2-Dimethoxy-2-phenyl-acetophenon und besonders bevorzugt α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin, verwendet. Geeignet sind auch Norrish-Typ-I-Photoinitiatoren, vor allem Acyl- der Bisacylphosphinoxide, und besonders geeignet sind Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium (MBDEGe). Dabei lassen sich auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Gemäß einer bevorzugten Ausführungsform enthalten die erfindungsgemäßen Dentalwerkstoffe zusätzlich mindestens einen organischen oder vorzugsweise anorganischen Füllstoff. Bevorzugt sind faserförmige und insbesondere partikuläre Füllstoffe.

Als faserförmige Füllstoffe sind Nanofasern, Glasfasern, Polyamidfasern und Kohlenstofffasern bevorzugt. Unter Nanofasern werden Fasern mit einer Länge von weniger als 100 nm verstanden. Faserförmige Füllstoffe eignen sich besonders zur Herstellung von Verbundmaterialien.

Bevorzugte anorganische Füllstoffe sind amorphe, kugelförmige, nanopartikuläre Füllstoffe auf der Basis von Oxiden, wie pyrogene Kieselsäure oder Fällungskieselsäure, ZrO₂ und TiO₂ oder Mischoxide aus SiO₂, ZrO₂ und/oder TiO₂, mikrofeine Füllstoffe, wie Quarz-, Glaskeramik-, oder Glaspulver, und röntgenopake Füllstoffe, wie Ytterbiumtrifluorid, nanopartikuläres Tantal(V)-oxid oder Bariumsulfat. Das Ytterbiumtrifluorid hat vorzugsweise eine Partikelgröße von 200 bis 800 nm.

Bevorzugte Glaspulver sind röntgenopake Glaspulver, insbesondere von Barium- oder Strontiumaluminiumsilikatgläsern. Glaspulver werden durch Mahlen gewonnen, sind rein anorganischer Natur und bestehen meist aus splitterförmigen Teilen.

Partikuläre Füllstoffe weisen vorzugsweise eine Partikelgröße von 0,01 bis 15 µm auf. Nanopartikuläre Füllstoffe haben vorzugsweise eine Partikelgröße von 10 bis 100 nm und mikrofeine Füllstoffe vorzugsweise eine Partikelgröße von 0,2 bis 5 µm. Röntgenopake Füllstoffe haben, soweit es sich nicht um nanopartikuläre Füllstoffe handelt, vorzugsweise eine Partikelgröße von 0,2 bis 5 µm.

Wenn nicht anders angegeben, handelt es sich bei allen Partikelgrößen um gewichtsmittlere Partikelgrößen (D50-Werte), wobei die Partikelgrößenbestimmung im Bereich von 0,1 µm bis 1000 µm vorzugsweise mittels statischer Lichtstreuung erfolgt, beispielsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Partikelgrößen kleiner als 0,1 µm können auch anhand von REM- oder TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Zur Verbesserung des Verbundes zwischen den Füllstoffpartikeln und der vernetzten Polymerisationsmatrix können SiO₂-basierende Füllstoffe mit (Meth)acrylat-funktionalisierten Silanen oberflächenmodifiziert werden. Ein Beispiel für solche Silane ist 3-(Meth)acryloyloxypropyltrimethoxysilan. Zur Oberflächenmodifizierung von nichtsilikatischen Füllstoffen wie z.B. von ZrO₂ oder TiO₂ können auch funktionalisierte saure Phosphate, wie z.B. 10-(Meth)acryloyloxydecyldihydrogenphosphat eingesetzt werden.

Der Füllungsgrad richtet sich nach dem gewünschten Anwendungszweck. Füllungskomposite haben vorzugsweise einen Füllstoffgehalt von 75-90 Gew.-% und Kompositzemente von 50-75 Gew.-%.

Bevorzugte Dentalwerkstoffe enthalten damit neben mindestens einer Verbindung der Formel I, II oder III zusätzlich mindestens ein radikalisch polymerisierbares Monomer, insbesondere mindestens ein multifunktionelles (Meth)acrylat oder eine Mischungen von mono- und multifunktionellen (Meth)acrylaten, mindestens einen Initiator für die radikalische Polymerisation und vorzugsweise auch mindestens einen Füllstoff.

Gegebenenfalls können die erfindungsgemäß eingesetzten Zusammensetzungen weitere Additive enthalten, vor allem Stabilisatoren, Farbmittel, mikrobiozide Wirkstoffe, fluoridionenabgebende Additive, Treibmittel, optische Aufheller, Weichmacher und/oder UV-Absorber.

Die erfindungsgemäßen Dentalmaterialien enthalten vorzugsweise 0,1 bis 30 Gew.-%, bevorzugt 0,2 bis 25 Gew.-% und besonders bevorzugt 0,5 bis 20 Gew.-% mindestens einer Verbindung der allgemeinen Formel I, II und/oder III, vorzugsweise I.

Zusätzlich enthalten die Materialien vorzugsweise auch 0,01 bis 5,0 Gew.-%, bevorzugt 0,1 bis 5,0 Gew.-% und besonders bevorzugt 0,1 bis 3,0 Gew.-% Initiator(en) für die radikalische Polymerisation, besonders bevorzugt einen Photoinitiator, und besonders bevorzugt auch 0 bis 99,9 Gew.-%, bevorzugt 10 bis 95 Gew.-% und besonders bevorzugt 15 bis 95 Gew.-% multifunktionelle (Meth)acrylat(e).

Weiterhin enthalten die erfindungsgemäßen Dentalwerkstoffe vorzugsweise 0 bis 90 Gew.-%, bevorzugt 0 bis 85 Gew.-% und besonders bevorzugt 0 bis 82 Gew.-% Füllstoff (e), wobei der Füllstoffgehalt wie oben beschrieben auf die geplante Verwendung der Werkstoffe abgestimmt wird.

Außerdem enthalten die erfindungsgemäßen Dentalwerkstoffe vorzugsweise 0 bis 70 Gew.-%, bevorzugt 0,1 bis 70 Gew.-%, besonders bevorzugt 0,1 bis 60 Gew.-% sonstige Additiv(e).

Dabei sind erfindungsgemäß Dentalmaterialien besonders bevorzugt, welche die folgenden Komponenten enthalten:
(a) 0,1 bis 30 Gew.-%, bevorzugt 0,2 bis 25 Gew.-% und besonders bevorzugt 0,5 bis 20 Gew.-% mindestens einer Verbindung der allgemeinen Formel I, II oder III, vorzugsweise I,
(b) 10 bis 99,1 Gew.-%, bevorzugt 10 bis 95 Gew.-% und besonders bevorzugt 15 bis 95 Gew.-% mindestens eines weiteren radikalisch polymerisierbaren Monomers, vorzugsweise mindestens eines multifunktionellen (Meth)acrylats,
(c) 0,01 bis 5,0 Gew.-%, bevorzugt 0,1 bis 5,0 Gew.-% und besonders bevorzugt 0,1 bis 3,0 Gew.-% mindestens eines Initiators für die radikalische Polymerisation,
(d) 0 bis 90 Gew.-%, bevorzugt 0 bis 85 Gew.-% und besonders bevorzugt 0 bis 82 Gew.-% mindestens eines Füllstoffs, und
(e) 0 bis 70 Gew.-%, bevorzugt 0,1 bis 70 Gew.-% und besonders bevorzugt 0,1 bis 60 Gew.-% eines oder mehrerer Additive.

Wenn nicht anders angegeben beziehen sich alle Mengenangaben auf die Gesamtmasse der Materialien. Die einzelnen Mengenbereiche können separat gewählt werden.

Besonders bevorzugt sind solche Dentalwerkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind. Besonders bevorzugt sind au-βerdem Werkstoffe, die neben der Verbindung der Formel I, II und/oder III keine flüchtigen Mercaptane enthalten, d.h. Mercaptane, die einen typischen Mercaptangeruch aufweisen. Ganz besonders bevorzugt sind Zusammensetzungen, die keine weiteren Mercaptane und vorzugsweise auch keine anderen Schwefelverbindungen enthalten.

Die erfindungsgemäßen Dentalwerkstoffe eignen sich besonders als dentale Zemente, Füllungskomposite und Verblendmaterialien sowie als Materialien zur Herstellung von Inlays, Onlays, Kronen und Brücken. Sie haben ähnliche mechanische Eigenschaften (Biegefestigkeit und E-Modul) wie auf Dimethacrylaten basierende Materialien und weisen eine vergleichbare Polymerisationsgeschwindigkeit auf, zeichnen sich jedoch durch eine verringerte Polymerisationsschrumpfungsspannung (PKS), eine verbesserte Schlagzähigkeit und geringen Eigengeruch aus.

Zusammensetzungen, die sich besonders als dentale Zemente eignen, enthalten bevorzugt:
(a) 0,1 bis 20 Gew.-%, bevorzugt 0,2 bis 15 Gew.-% besonders bevorzugt 0,5 bis 10 Gew.-% mindestens eines Urethanallylsulfids der allgemeinen Formel I, II und/oder III,
(b) 5 bis 50 Gew.-% , bevorzugt 10 bis 50 Gew.-% und besonders bevorzugt 20 bis 40 Gew.-% mindestens eines weiteren radikalisch polymerisierbaren Monomers,
(c) 0,01 bis 5,0 %, bevorzugt 0,1 bis 5,0 Gew.-% und besonders bevorzugt 0,2 bis 3,0 Gew.-% mindestens eines Initiators für die radikalische Polymerisation,
(d) 10 bis 70 Gew.-%, bevorzugt 20 bis 70 Gew.-% und besonders bevorzugt 30 bis 70 Gew.-% mindestens eines Füllstoffs und
(e) 0 bis 5 Gew.-%, bevorzugt 0,1 bis 5,0 Gew.-% und besonders bevorzugt 0,2 bis 4,0 Gew.-% eines oder mehrerer Additive.

Zusammensetzungen, die sich besonders als dentale Komposite eignen, enthalten bevorzugt:
(a) 0,1 bis 20 Gew.-%, bevorzugt 0,2 bis 15 Gew.-% besonders bevorzugt 0,5 bis 8 Gew.-% mindestens eines Urethanallylsulfids der allgemeinen Formel I, II und/oder III,
(b) 10 bis 50 Gew.-%, bevorzugt 15 bis 40 Gew.-% und besonders bevorzugt 15 bis 30 Gew.-% mindestens eines weiteren radikalisch polymerisierbaren Monomers,
(c) 0,01 bis 5,0 Gew.-%, bevorzugt 0,1 bis 5,0 Gew.-% und besonders bevorzugt 0,1 bis 0,3 Gew.-% mindestens eines Initiators für die radikalische Polymerisation,
(d) 10 bis 85 Gew.-%, bevorzugt 20 bis 85 Gew.-% und besonders bevorzugt 30 bis 85 Gew.-% mindestens eines Füllstoffs und
(e) 0 bis 5 Gew.-%, bevorzugt 0,1 bis 5,0 Gew.-% und besonders bevorzugt 0,2 bis 3,0 Gew.-% eines oder mehrerer Additive.

Die Dentalwerkstoffe eignen sich primär zur intraoralen Anwendung durch den Zahnarzt zur Restauration geschädigter Zähne (therapeutische Anwendung), z.B. als dentale Zemente, Füllungskomposite und Verblendmaterialien. Sie können aber auch extraoral eingesetzt werden, beispielsweise bei der Herstellung oder Reparatur von Dentalrestaurationen, wie Inlays, Onlays, Kronen und Brücken (nicht therapeutische Anwendung). Die Erfindung wird im Folgenden anhand von Ausführungsbeispielen näher erläutert.

### Ausführungsbeispiele

### Beispiel 1:

### Synthese von 2-[2-(Hydroxyethyl)thiomethyl]acrylsäureethylester (1)

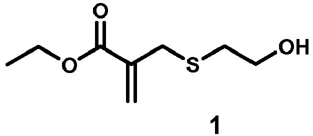

Eine Lösung von 2-Chlormethylacrylsäureethylester (7,43 g, 50,0 mmol) in Dichlormethan (50 ml) wurde bei 0°C zu einer Lösung von 2-Mercaptoethanol (3,91 g, 50,0 mmol) und Triethylamin (5,06 g, 50,0 mmol) in Dichlormethan (150 ml) zugetropft. Das Reaktionsgemisch wurde 20 h bei Umgebungstemperatur gerührt und anschliessend mit Wasser (2x 100 m) und gesättigter wässriger Natriumchlorid-Lösung (3x 100 ml) gewaschen, über wasserfreiem Natriumsulfat getrocknet und über Kieselgel filtriert. Das Filtrat wurde am Rotationsverdampfer eingeengt und man erhielt 8,98 g (47,2 mmol;94%) eines farblosen Öls.

¹H-NMR (CDCl₃, 400 MHz) : δ = 6,22 (d, 1H; *J* = 1,0 Hz; =CH), 5,69 (d, 1H; *J* = 1.0 Hz; =CH), 4,24 (q, 2H; *J* = 7,1 Hz; O-CH₂), 3,74 (m, 2H; CH₂OH), 3,43 (s, 2H; CH₂-S), 2,96 (m, 1H; OH), 2,68 (t, 2H; *J* = 6, 1 Hz; S-CH₂), 1,32 (t, 3H; *J* = 7.1 Hz; CH₃). ¹³C-NMR (CDCl₃, 100.6 MHz) : δ = 166,0 (C=O) , 136,8 (=C), 126,0 (=CH₂), 60,9 (O-CH₂), 60,5 (O-CH₂), 34,2 (S-CH₂), 32,1 (S-CH₂), 13,9 (CH₃).

### Beispiel 2:

### 2-(12-Methyl-6,11-dioxo-5,10-dioxa-2-thia-7-azatridec-12-en-1-yl)acrylsäureethylester (2)

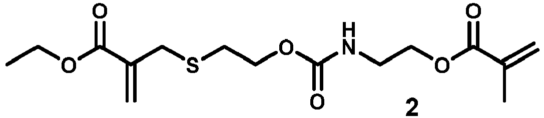

2-[2-(Hydroxyethyl)thiomethyl]acrylsäureethylester (60,0 mmol) wurde in Dichlormethan gelöst und Dibutylzinndilaurat (10 mg) wurde zugegeben. Das Isocyanat 2-Isocyanatoethylmethacrylat (60,0 mmol) wurde zugetropft und das Reaktionsgemisch wurde so lange bei Umgebungstemperatur gerührt, bis mittels IR-Spektroskopie keine N=C=O-Bande mehr detektierbar war (4h-24h). Die Reaktionslösung wurde mit Natronlauge (1*N*), Salzsäure (1*N*) und Wasser gewaschen, über wasserfreiem Natriumsulfat getrocknet, filtriert und am Rotationverdampfer eingeengt. Das Rohprodukt wurde mittels Säulenchromatographie gereinigt und man erhielt die reine Verbindung 2 als gelbliches Öl (Ausbeute >70%) .

¹H-NMR (CDCl₃, 400 MHz) : δ = 6,22 (d, 1H; *J* = 0,6 Hz; =CH), 6,13 (s, 1H; =CH), 5,69 (s, 1H; =CH), 5,60 (t, 1H; *J* = 1,5 Hz; =CH), 5,21 (s, 1H; NH), 4,27-4,18 (m, 6H; O-CH₂), 3,52-3,47 (m, 2H; N-CH₂) , 3,42 (s, 2H; CH₂-S) , 2,69 (t, 2H; *J* = 6,7 Hz; S-CH₂) , 1,95 (s, 3H; CH₃) , 1,32 (t, 3H; *J* = 7,1 Hz; CH₃). ¹³C-NMR (CDCl₃, 100.6 MHz) : δ = 167,1 (C=O) , 166,0 (C=O) , 156,1 (C=O) , 136,8 (=C), 135,8 (=C), 126,1 (=CH₂), 125,9 (=CH₂), 63,5 (O-CH₂), 60,9 (O-CH₂), 40,0 (N-CH₂), 32,6 (S-CH₂), 30,0 (S-CH₂), 18,2 (CH₃), 14,0 (CH₃).

### Beispiel 3:

### 2-{2-[(Hexylcarbamoyloxy)ethyl]thiomethyl}acrylsäureethylester (3)

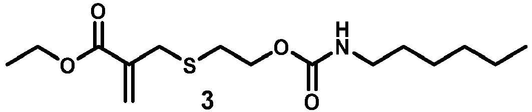

Analog zu Beispiel 2 wurden als Isocyanat 60 mmol n-Hexylisocyanat eingesetzt und 3 als farblose Flüssigkeit erhalten:

¹H-NMR (CDCl₃, 400 MHz): δ = 6,22 (s, 1H; =CH), 5,69 (s, 1H; =CH), 4,81 (s, 1H; NH), 4,24 (q, 2H; J = 7,2 Hz; O-CH₂), 4,19 (t, 2H; *J* = 6,7 Hz; O-CH₂), 3,43 (s, 2H; CH₂-S) , 3,16 (m, 2H; N-CH₂) , 2,68 (t, 2H; J = 6.7 Hz; S-CH₂), 1,48 (m, 2H; CH₂) , 1,31 (m, 9H; CH₂-CH₃) , 0,88 (t, 3H; *J* = 6,7 Hz; CH₃).

¹³C-NMR (CDCl₃, 100,6 MHz) : δ = 166,0 (C=O) , 156,1 (C=O) , 136,8 (=C), 126,1 (=CH₂), 63,3 (O-CH₂), 61,0 (O-CH₂), 41,0 (N-CH₂), 32,7 (CH₂), 31,4 (CH₂), 30,2 (CH₂), 29,8 (CH₂), 26,2 (CH₂), 22,5 (CH₂), 14,1 (CH₃), 13,9 (CH₃).

### Beispiel 4:

### 2-{2-[(Dodecylcarbamoyloxy)ethyl]thiomethyl}acrylsäureethylester (4)

Analog zu Beispiel 2 wurden als Isocyanat 60 mmol Dodecylisocyanat eingesetzt und 4 als farblose Flüssigkeit erhalten:

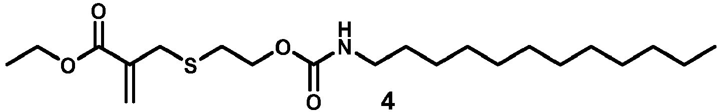

¹H-NMR (CDCl₃, 400 MHz) : δ = 6,22 (d, 1H; *J* = 0,7 Hz; =CH), 5,69 (s, 1H; =CH), 4,73 (s, 1H; NH), 4,24 (q, 2H; *J* = 7,1 Hz; O-CH₂), 4,19 (t, 2H; *J* = 6,7 Hz; O-CH₂), 3,43 (s, 2H; CH₂-S) , 3,16 (m, 2H; N-CH₂), 2,68 (t, 2H; *J* = 6,7 Hz; S-CH₂), 1,49 (m, 2H; CH₂) , 1, 33-1, 24 (m, 21H; CH₂-CH₃) , 0,88 (t, 3H; *J* = 6,8 Hz; CH₃) .

¹³C-NMR (CDCl₃, 100,6 MHz) : δ = 166,1 (C=O) , 156,2 (C=O) , 136,9 (=C), 126,1 (=CH₂), 63,3 (O-CH₂), 61,0 (O-CH₂), 41,0 (N-CH₂), 32,7 (CH₂), 31,9 (CH₂), 30,2 (CH₂), 29,9 (CH₂), 29,6 (CH₂), 29,6 (CH₂), 29,5 (CH₂), 29,5 (CH₂), 29,3 (CH₂), 29,2 (CH₂), 26,7 (CH₂), 22,6 (CH₂), 14,1 (CH₃), 14,1 (CH₃).

### Beispiel 5:

### 2-{2-[(Benzylcarbamoyloxy)ethyl]thiomethyl}acrylsäureethylester (5)

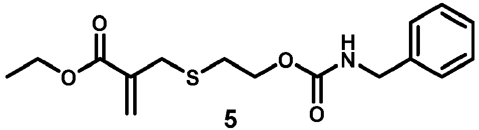

Analog zu Beispiel 2 wurden als Isocyanat 60 mmol Benzylisocyanat eingesetzt und **5** als farblose Flüssigkeit erhalten: ¹H-NMR (CDCl₃, 400 MHz) : δ = 7,35-7,25 (m, 5H; Ar-H), 6,21 (s, 1H; =CH), 5,67 (s, 1H; =CH), 5,23 (s, 1H; NH), 4,35 (d, 2H; *J* = 6,0 Hz; N-CH₂) , 4,22 (m, 4H; O-CH₂), 3,41 (s, 2H; CH₂-S), 2,68 (t, 2H; *J* = 6.8 Hz; S-CH₂), 1,30 (t, 3H; *J* = 7,2 Hz; CH₃). ¹³C-NMR (CDCl₃, 100,6 MHz): δ = 166,1 (C=O) , 156,2 (C=O) , 138,3 (Ar-C), 136,8 (=C), 128,6 (Ar-CH), 127,4 (Ar-CH), 126,1 (=CH₂), 63,5 (O-CH₂), 61,0 (O-CH₂), 44,9 (N-CH₂), 32,7 (S-CH₂), 30,1 (S-CH₂), 14,1 (CH₃).

### Beispiel 6:

### 11,13,13-Trimethyl-2,23-dimethylen-8,17-dioxo-7,18-dioxa-4,21-dithia-9,16-diazatetracosandionsäurediethylester, Isomerengemisch (6)

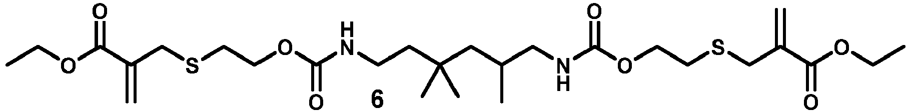

Analog zu Beispiel 2 wurden als Isocyanat 30 mmol 2,2,4-Trimethylhexamethylendiisocyanat eingesetzt und **6** als farbloser Feststoff erhalten:
¹H-NMR (CDCl₃, 400 MHz): δ = 6,22 (s, 2H; =CH), 5,69 (s, 2H; =CH), 5,10-4,71 (m, 2H; NH), 4,30-4,14 (m, 8H; O-CH₂), 3,43 (s, 4H; CH₂-S), 3,23-2,84 (m, 4H; N-CH₂), 2,68 (t, 4H; *J* = 7.1 Hz; S-CH₂), 1,64-1,23 (m, 11H, CH, CH₂, CH₃), 0,97-0,85 (m, 9H; CH₃) .

¹³C-NMR (CDCl₃, 100,6 MHz): δ = 166,1 (C=O), 156,5 (C=O), 156,4 (C=O), 156,2 (C=O), 156,1 (C=O), 136,9 (=C), 126,1 (=CH₂), 63,4 (O-CH₂), 61,0 (O-CH₂), 51,3 (CH₂), 48,5 (CH₂), 46,4 (CH₂), 45,9 (CH₂), 41,9 (CH₂), 39,3 (CH₂), 39,0 (CH₂), 37,2 (CH₂), 35,0 (CH₂), 32,8 (C), 32,7 (CH₂), 30,2 (CH₂), 29,4 (CH₃), 27,4 (CH₃), 27,4 (CH₃), 26,2 (CH), 25,4 (CH), 25,1 (CH₃), 22,3 (CH₃), 20,5 (CH₃), 14,1 (CH₃).

### Beispiel 7:

### 1,3-Bis(2-methyl-10-methylen-4,11-dioxo-5,12-dioxa-8-thia-3-azatetradecan-2-yl)benzol, Isomerengemisch (7)

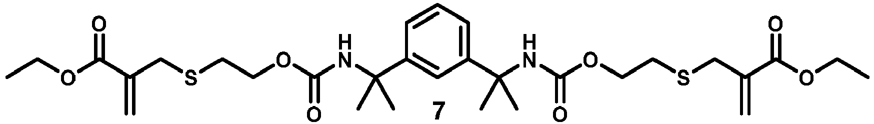

Analog zu Beispiel 2 wurden als Isocyanat 30 mmol 1,3-Bis(1-isocyanato-1-methylethyl)benzol eingesetzt und **7** als farbloser Feststoff erhalten:
¹H-NMR (CDCl₃, 400 MHz): δ = 7,41 (s, 1H; Ar-H), 7,32-7,25 (m, 3H; Ar-H), 6,20 (s, 2H; =CH), 5,65 (s, 2H; =CH), 5,21 (s, 2H; NH), 4,23 (q, 4H; *J* = 7,1 Hz; O-CH₂), 4,17-4,07 (m, 4H; O-CH₂) , 3,41 (s, 4H; CH₂-S), 2,66 (s, 4H; S-CH₂), 1,66 (s, 12H; CH₃) , 1,31 (t, 6H; *J* = 7,2 Hz; CH₃).

¹³C-NMR (CDCl₃, 100,6 MHz) : δ = 166,0 (C=O) , 154,2 (C=O) , 146,9 (Ar-C), 136,8 (=C), 128,3 (Ar-CH), 126,1 (=CH₂), 123,2 (Ar-CH), 121,2 (Ar-CH), 62,8 (O-CH₂), 61,0 (O-CH₂), 55,4 (N-C), 32,6 (S-CH₂), 30,2 (S-CH₂), 29.2 (CH₃), 14,1 (CH₃).

### Beispiel 8:

### 2-(Dodecylthiomethyl)acrylsäureethylester (8, Referenzverbindung)

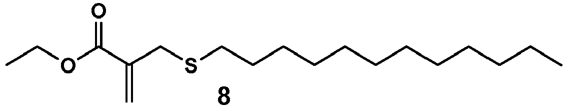

Eine Lösung von 2-Chlormethylacrylsäureethylester (14,88 g, 0,10 mol) in Tetrahydrofuran (50 ml) wurde bei 0 °C zu einer Lösung von 1-Dodecanthiol (20,24 g, 0,10 mol) und Triethylamin (10,12 g, 0,10 mol) in Tetrahydrofuran (50 ml) zugetropft. Das Reaktionsgemisch wurde 20 h bei Umgebungstemperatur gerührt, dann wurde die Suspension filtriert. Das Filtrat wurde mit Wasser (150 ml) und n-Heptan (150 ml) versetzt und die Phasen wurden getrennt. Die Wasserphase wurde mit n-Heptan (2x 150 ml) extrahiert. Die vereinigten organischen Phasen wurden über wasserfreiem Natriumsulfat getrocknet und über Kieselgel filtriert. Das Filtrat wurde am Rotationsverdampfer eingeengt und man erhielt 22,45 g (71,4 mmol; 71%) einer leicht gelblichen Flüssigkeit.

¹H-NMR (CDCl₃, 400 MHz) : δ = 6,20 (d, 1H; *J* = 0,9 Hz; =CH), 5,63 (d, 1H; *J* = 0,9 Hz; =CH), 4,24 (q, 2H; *J* = 7,1 Hz; O-CH₂), 3,38 (d, 2H; *J* = 0,6 Hz; CH₂-S) , 2,45 (t, 2H; *J* = 7,5 Hz; S-CH₂) , 1,56 (m, 2H; CH₂), 1,44-1,16 (m, 24H; CH₂), 0,88 (t, 3H; *J* = 6,8 Hz; CH₃).

¹³C-NMR (CDCl₃, 100,6 MHz) : δ = 166,2 (C=O) , 137,3 (=C), 125,4 (=CH₂), 60,9 (O-CH₂), 32,7 (CH₂), 31,9 (CH₂), 31,6 (CH₂), 29,6 (CH₂), 29,5 (CH₂), 29,3 (CH₂), 29,2 (CH₂), 28,9 (CH₂), 22,6 (CH₂), 14,1 (CH₃).

### Beispiel 9:

### 2-(Methylenpropan-1,3-bis(2-[2-methacryloyloxyethylcarbamoyl]-ethylsulfid) (V-783, Referenzverbindung)

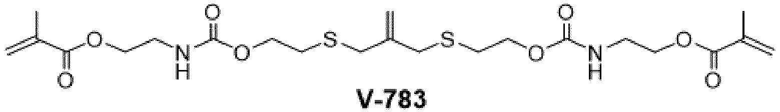

### 1. Stufe: 2-Methylenpropan-1,3-bis(2-hydroxyethylsulfid)

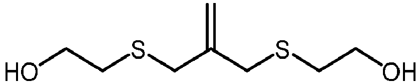

2-Mercaptoethanol (32,03 g; 0,41 mol), 3-Chlor-2-chlormethyl-1-propen (25,00 g; 0,20 mol) und 18-Krone-6 (7,95 g; 30,0 mmol) wurden in 2-Butanon (200 ml) gelöst. Kaliumcarbonat (67,72 g; 0,49 mol) wurde zugegeben und das Reaktionsgemisch wurde 20 h am Rückfluss erhitzt. Nach dem Abkühlen wurde die Suspension mit Ethylacetat (200 ml) verdünnt und über Kieselgel filtriert. Das Filtrat wurde am Rotationsverdampfer eingeengt. Das Rohprodukt wurde mittels Säulenchromatographie gereinigt (SiO₂, n-Hexan/Ethylacetat) und man erhielt 32,34 g (0.155 mol; 78%) einer gelblichen Flüssigkeit.

¹H-NMR (CDCl₃, 400 MHz): δ = 5,04 (s, 2H; =CH), 3,71 (m, 4H; O-CH₂) , 3,43 (m, 2H; OH), 3,32 (s, 4H; S-CH₂) , 2,62 (t, 4H; *J* = 6,3 Hz; CH₂-S) . ¹³C-NMR (CDCl₃, 100,6 MHz): δ = 140,2 (=C), 115,9 (=CH₂), 60,2 (O-CH₂), 34,7 (S-CH₂), 33,4 (CH₂-S).

### 2. Stufe: 2-Methylenpropan-1,3-bis(2-[2-methacryloyloxyethylcarbamoyl]ethylsulfid) (V-783)

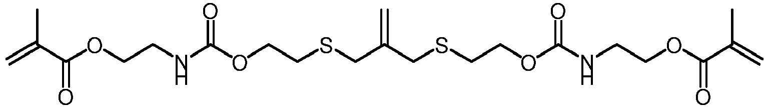

2-Methylenpropan-1,3-bis(2-hydroxyethylsulfid) der 1. Stufe (7,89 g; 37,9 mmol) und Dibutylzinndilaureat (0,244 g; 2,0 mmol) wurden in Aceton (100 ml) gelöst. Isocyanatoethylmethacrylat (11,75 g; 75,7 mmol) und das Reaktionsgemisch wurde bei Umgebungstemperatur gerührt. Nach 3 h wurde Methanol (20 ml) zugegeben und die Lösung wurde am Rotationsverdampfer eingeengt. Der Rückstand wurde mit n-Hexan (100 ml) versetzt und die Suspension wurde 24 h bei RT gerührt. Die Suspension wurde filtriert. Der Filtrationsrückstand wurde mit n-Hexan (100 ml) gewaschen und im Vakuumtrockenschrank getrocknet. Man erhielt 18,57 g (35,8 mmol; 94%) eines weißen Feststoffs.

¹H-NMR (CDCl₃, 400 MHz) : δ = 6,13 (s, 2H; =CH), 5,60 (m, 2H; =CH), 5,29 (br s, 2H; NH), 5,05 (s, 2H; =CH), 4,25 - 4,16 (m, 8H; CH₂-O), 3,50 (m, 4H; CH₂-N), 3,32 (s, 4H; S-CH₂), 2,65 (t, 4H; *J* = 6,7 Hz; CH₂-S) , 1,95 (s, 6H; CH₃).

¹³C-NMR (CDCl₃, 100,6 MHz) : δ = 167,1 (C=O) , 156,0 (C=O) , 140,1 (=C), 135,8 (=C), 125,9 (=CH₂), 116,3 (=CH₂), 63,4 (O-CH₂), 63,3 (O-CH₂), 40,0 (N-CH₂), 35,1 (S-CH₂), 29,7 (CH₂-S), 18,1 (CH₃).

### Beispiel 10:

### Herstellung von Füllungskompositen mit erfindungsgemäßen Übertragungsreagenzien (Angaben in Gew. -%)

Es wurde eine Dimethacrylatmischung bestehend aus 42,19 % Bis-GMA, 37,57 % UDMA und 20,33 % D₃MA hergestellt. Zu 83,85 g dieser Dimethacrylatmischung wurden folgende Komponenten zugegeben: 15 g des jeweiligen Allylsulfids sowie die Photoinitiatorkomponenten CQ (0,15 g), EDMAB (0,4 g) und Lucirin TPO (0,4 g, 2,4,6-Trimethylbenzoyldiphenylphosphinoxid).

Mit der so hergestellten Photopolymerisationsharzmischung wurden in einem Kneter der Firma Linden Komposite hergestellt. Dazu wurden zu 33,63 g Photopolymerisationsharzmischung folgende Komponenten zugegeben: 52,21 g eines silanisierten Ba-Al-Borosilikatglasfüllers (GM27884, Fa. Schott) mit einer mittleren Partikelgröße von 1,0 µm, 4,02 g silanisierter Ba-Ca-Al-Fluorosilikatglasfüller (G018-056, Fa. Schott) mit einer mittleren Partikelgröße von 1,0 µm, 4,02 g silanisiertes SiO₂-ZrO₂-Mischoxid mit einer mittleren Partikelgröße von 1,2 µm (Sphärosil, Fa. Transparent Materials), 0,80 g der pyrogenen Kieselsäure OX-50 (Fa. Evonik), 2,51 g YbF₃ (Ytterbiumtrifluorid, mittleren Partikelgröße von 0,2 µm, Fa. Auer Remy) sowie 2,81 g Rheologiemodifizierungsmittel (InTen-S-Bentone).

Nach der Kompositherstellung wurden Prüfkörper präpariert, die 2 mal 3 Minuten mit einer dentalen Lichtquelle (Spectramat, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit (BF) und des Biege-E-Moduls (BM). Zur Bestimmung der Polymerisationsschrumpfungskraft (PKS) wurden die Proben einseitig auf einem silanisierten Objektträger fixiert und mittels eines mit Haftvermittler (Monobond, Fa. Ivoclar Vivadent AG) behandelten Stahlstempel (d = 10 mm) mit einer Zwick Universalprüfmaschine verbunden. Nach dem Einstellen der Schichtdicke (0,8 mm) und dem Entfernen von Überschüssen wurde die Messung gestartet. Die Belichtung (Bluephase 20i, High Power, 10 s) erfolgt durch den Objektträger und startet 120 s nach Messbeginn. Während insgesamt 10 Minuten wurde die Kraftänderung bei konstant gehaltener Traversenposition registriert. Die resultierenden Messwerte sind in der Tabelle 1 zusammengestellt

Die Ergebnisse belegen, dass sich die Komposite mit erfindungsgemäßen Urethanallylverbindungen, d.h. die Komposite K-2 bis K-4 und K-6, gegenüber dem Referenzkomposit K-5 (ohne Allylsulfid) durch eine signifikant reduzierte Polymerisationsschrumpfungskraft auszeichnen und vergleichbare mechanischen Eigenschaften aufweisen. Demgegenüber zeigt das Komposit K-1 auf der Basis des aus dem Stand der Technik bekannten Allylsulfids **8** zwar eine deutlich reduzierte Polymerisationsschrumpfungskraft, jedoch sind die Biegefestigkeit und der Biege-E-Modul deutlich reduziert. Erstaunlicherweise bewirkt das bekannte Allylsulfid V-783 im Vergleich zu dem strukturähnlichen Allylsulfid **6** nur eine relativ geringe Verbesserung der Polymerisationsschrumpfungskraft.

**Tabelle 1: Eigenschaften der Komposite K-1 bis K-7**

| | **K-1*)** | **K-2** | **K-3** | **K-4** | **K-5*)** | **K-6** | **K-7*)** |
|---|---|---|---|---|---|---|---|
| Allylsulfid | **8** | **3** | **2** | **6** | - | **5** | **V-783** |
| BF (MPa) 24 h | 80,7 | 106,0 | 107,8 | 101,8 | 119,7 | 107,2 | 114,7 |
| BF (MPa) 24 h WL¹⁾ | 88,0 | 130,9 | 115,3 | 121,4 | 132,3 | 121,9 | 115,3 |
| BM (GPa) 24 h | 3,73 | 5,20 | 5,87 | 4,92 | 6,26 | 6,06 | 7,03 |
| BM (GPa) 24 h WL¹ | 3,90 | 6,87 | 6, 61 | 6,34 | 7,14 | 7,13 | 7,16 |
| PKS (N) nach 125 s | 7,0 | 13,8 | 16,1 | 10,1 | 39,8 | 10,2 | 27,7 |
| PKS (N) nach 600 s | 39,4 | 50,5 | 48,5 | 40,9 | 62,8 | 38,8 | 60,2 |

| | | | | | | | |
|---|---|---|---|---|---|---|---|
| ^{*)} Vergleichsbeispiel ¹⁾ WL = Wasserlagerung der Prüfkörper | | | | | | | |

## Patentansprüche

1. Radikalisch polymerisierbarer Dentalwerkstoff, der mindestens eine Verbindung der der Formeln I bis III enthält: 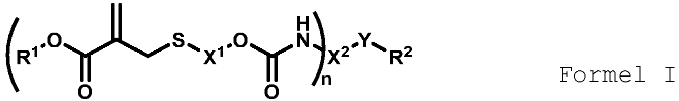 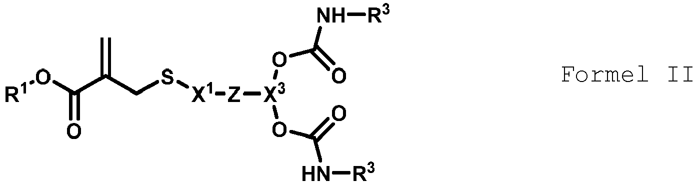 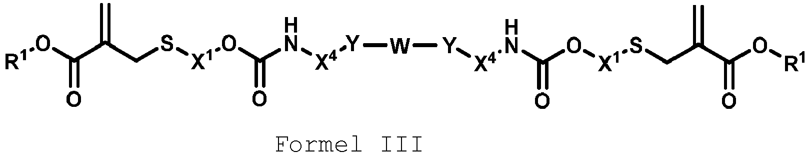 in denen die Variablen die folgenden Bedeutungen haben:
R¹ Wasserstoff, ein aliphatischer linearer oder verzweigter C₁-C₁₅-Alkyl-Rest, der durch ein oder mehrere, vorzugsweise 1 bis 3, O oder S unterbrochen und der substituiert oder unsubstituiert sein kann, Benzyl oder Phenyl;
R² Wasserstoff, ein aliphatischer linearer oder verzweigter C₁-C₁₅-Alkyl-Rest, der durch ein oder mehrere, vorzugsweise 1 bis 3, O oder S unterbrochen und der substituiert oder unsubstituiert sein kann, Benzyl, ein aromatischer C₆-C₁₈-Rest, der substituiert oder unsubstituiert sein kann, oder eine (Meth)acryloyloxy-Gruppe;
R³ ein aliphatischer linearer oder verzweigter C₁-C₁₅-Alkyl-Rest, der durch ein oder mehrere O unterbrochen und der substituiert oder unsubstituiert sein kann, Benzyl, oder ein aromatischer C₆-C₁₈-Rest, der substituiert oder unsubstituiert sein kann;
X¹ ein linearer oder verzweigter aliphatischer C₁-C₁₅-Alkylen-Rest, der durch ein oder mehrere O oder S unterbrochen sein kann, oder ein cycloaliphatischer C₆-C₁₆-Rest;
X² ein (n+1)-wertiger organischer C₁-C₂₀-Rest, der durch ein oder mehrere O oder S unterbrochen sein kann, ein cycloaliphatischer C₅-C₂₀-Rest, ein aromatischer C₆-C₂₀-Rest oder eine Isocyanursäurerest gemäß der folgenden Formel: 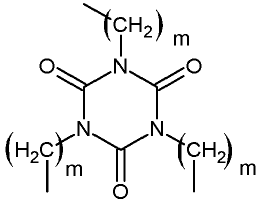
X³ ein 3-wertiger linearer oder verzweigter aliphatischer C₃-C₂₀-Rest;
X⁴ ein 2-wertiger organischer C₂-C₃₀-Rest, der durch eine oder mehrere Methylgruppen substituiert sein kann oder unsubstituiert ist;
Y entfällt oder eine Ether- (-O-), Ester- (-COO- oder -OOC-), Urethan- (-NR⁴-CO-O- oder -O-CO-NR⁴-) oder Amid-Gruppe (-CONR⁴- oder -NR⁴-CO-), wobei R⁴ jeweils für H oder einen C₁-C₃-Alkyl-Rest steht;
Z entfällt oder eine Ether- (-O-), Ester- (-COO- oder -OOC-), oder Amid-Gruppe (-CONR⁵- oder -NR⁵-CO-), wobei R⁵ für H oder einen C₁-C₃-Alkyl-Rest steht;
W eine oligomere Polyester-, Polyether- oder Polythioethergruppe;
m 1, 2, 3, 4, 5 oder 6;
n 1, 2, 3 oder 4,
wobei dann, wenn X² ein Isocyanursäurerest ist, Y und R² entfallen und n gleich 3 ist.

2. Dentalwerkstoff nach Anspruch 1, der mindestens ein zusätzliches radikalisch polymerisierbares Monomer und vorzugsweise auch mindestens einen Initiator für die radikalische Polymerisation enthält.

3. Dentalwerkstoff nach Anspruch 1 oder 2, wobei die Variablen die folgenden Bedeutungen haben:
R¹ Wasserstoff, ein aliphatischer linearer oder verzweigter C₁-C₁₀-Alkyl-Rest, der durch ein oder mehrere, vorzugsweise 1 bis 2, O unterbrochen sein kann und der einen oder mehrere, vorzugsweise 1 bis 3, Substituenten tragen kann, die vorzugsweise aus -CH₃, -C₂H₅ und/oder polymerisationsfähigen (Meth)acryloyloxygruppen ausgewählt sind, oder unsubstituiert ist, Benzyl oder Phenyl;
R² Wasserstoff, ein aliphatischer linearer oder verzweigter C₁-C₁₀-Alkyl-Rest, der durch ein oder mehrere, vorzugsweise 1 bis 2, O unterbrochen sein kann und der ein oder mehrere, vorzugsweise 1 bis 3, Substituenten tragen kann, die vorzugsweise aus -CH₃, -C₂H₅ und/oder polymerisationsfähigen (Meth)acryloyloxygruppen ausgewählt sind, oder unsubstituiert ist, Benzyl, oder ein aromatischer C₆-C₁₂-Rest, der ein oder mehrere, vorzugsweise 1 bis 3, Substituenten tragen kann, die vorzugsweise aus -CH₃, -C₂H₅, und/oder eine polymerisationsfähigen (Meth)acryloyloxygruppen ausgewählt sind, oder unsubstituiert ist, oder eine (Meth)acryloyloxy-Gruppe;
R³ ein aliphatischer linearer oder verzweigter C₁-C₁₀-Alkyl-Rest, der ein oder mehrere, vorzugsweise 1 bis 3, Substituenten tragen kann, die vorzugsweise aus -CH₃, -C₂H₅, Halogen und/oder polymerisationsfähigen (Meth)acryloyloxygruppen ausgewählt sind, oder unsubstituiert ist, Benzyl, oder ein aromatischer C₆-C₁₈-Rest, der ein oder mehrere, vorzugsweise 1 bis 3, Substituenten tragen kann, die vorzugsweise aus -CH₃, -C₂H₅, polymerisationsfähigen Vinyl- und/oder (Meth)-acryloyloxygruppen ausgewählt sind, oder unsubstituiert ist;
X¹ ein linearer oder verzweigter aliphatischer C₁-C₁₀ Alkylen-Rest, der durch ein oder mehrere, vorzugsweise 1 bis 2, O unterbrochen sein kann, oder ein cycloaliphatischer C₆-C₁₂-Rest;
X² ein (n+1)-wertiger organischer C₁-C₁₅-Rest, vorzugsweise ein aliphatischer C₁-C₁₅-Rest, der durch ein oder mehrere, vorzugsweise 1 bis 2, O unterbrochen sein kann, ein cycloaliphatischer Rest oder ein aromatischer C₆-C₁₄-Rest;
X³ ein 3-wertiger linearer oder verzweigter aliphatischer C₃-C₁₀-Rest;
X⁴ ein 2-wertiger organischer C₂-C₂₀-Rest, vorzugsweise ein aliphatischer C₂-C₂₀-Rest, ein cycloaliphatischer C₅-C₁₀-Rest oder eine Kombination davon, der eine oder mehrere, vorzugsweise 1 bis 2, Methylgruppen als Substituenten tragen kann, oder unsubstituiert ist;
Y entfällt, oder eine Ester- oder Urethan-Gruppe;
Z entfällt oder eine Ether- oder Ester-Gruppe; W eine oligomere Polyester- oder Polyethergruppe und
n 1, 2 oder 3.

4. Dentalwerkstoff nach Anspruch 3, wobei die Variablen die folgenden Bedeutungen haben:
R¹ ein aliphatischer linearer oder verzweigter C₁-C₄-Alkyl-Rest oder Benzyl (Ph-CH₂-), ganz besonders bevorzugt Methyl oder Ethyl;
R² Wasserstoff oder eine (Meth)acryloyloxygruppe;
R³ ein linearer C₁-C₃-Alkyl-Rest, Benzyl (Ph-CH₂-), Phenyl (Ph-) oder p-Tolyl (H₃C-Ph-), wobei diese Reste jeweils durch eine (Meth)acryloyloxygruppe substituiert sein können;
X¹ ein linearer C₁-C₃ Alkylen-Rest;
X² ein (n+1)-wertiger, linearer oder verzweigter, aliphatischer C₂-C₁₂-Rest, der durch 1 bis 3 O unterbrochen sein kann, oder ein aromatischer C₆-C₁₂-Rest;
X³ ein 3-wertiger, linearer oder verzweigter, aliphatischer C₃-C₄-Rest; insbesondere -CH₂-CH(-)-CH₂-;
X⁴ ein 2-wertiger aliphatischer C₂-C₁₀-Rest oder ein cycloaliphatischer C₆-Rest, der mit 1 bis 3 Methylgruppen subsituiert sein kann;
Y entfällt, eine Ester- oder Urethan-Gruppe;
Z entfällt;
W eine oligomere Polyester- oder Polyethergruppe und
n 1, 2 oder 3, insbesondere 1 oder 2.

5. Dentalwerkstoff nach einem der Ansprüche 1 bis 4, wobei die bei den einzelnen Resten vorhandenen Substituenten jeweils aus C₁-C₃-Alkylgruppen, vorzugsweise CH₃- und/oder C₂H₅-, Halogen, OH, OCH₃ oder -O-COCH₃, einer polymerisationsfähigen Vinyl-, (Meth)acryloyloxy- und/oder (Meth)-acrylamidgruppen ausgewählt sind.

6. Dentalwerkstoff nach einem der Ansprüche 1 bis 5, der mindestens ein multifunktionelles (Meth)acrylat oder eine Mischungen von mono- und multifunktionellen (Meth)-acrylaten enthält.

7. Dentalwerkstoff nach einem der Ansprüche 1 bis 6, der mindestens einen Füllstoff enthält.

8. Dentalwerkstoff nach einem der Ansprüche 1 bis 7, der zusätzlich mindestens einen Photoinitiator für die radikalische Polymerisation enthält.

9. Dentalwerkstoff nach einem der Ansprüche 1 bis 8, der
(a) 0,1 bis 30 Gew.-%, bevorzugt 0,2 bis 25 Gew.-% und besonders bevorzugt 0,5 bis 20 Gew.-% mindestens einer Verbindung der allgemeinen Formel I, II oder III, vorzugsweise I,
(b) 10 bis 99,1 Gew.-%, bevorzugt 10 bis 95 Gew.-% und besonders bevorzugt 15 bis 95 Gew.-% mindestens eines weiteren radikalisch polymerisierbaren Monomers, vorzugsweise mindestens eines multifunktionellen (Meth)acrylats,
(c) 0,01 bis 5,0 Gew.-%, bevorzugt 0,1 bis 5,0 Gew.-% und besonders bevorzugt 0,1 bis 3,0 Gew.-% mindestens eines Initiators für die radikalische Polymerisation,
(d) 0 bis 90 Gew.-%, bevorzugt 0 bis 85 Gew.-% und besonders bevorzugt 0 bis 82 Gew.-% mindestens eines Füllstoffs, und
(e) 0 bis 70 Gew.-%, bevorzugt 0,1 bis 70 Gew.-% und besonders bevorzugt 0,1 bis 60 Gew.-% eines oder mehrerer Additive,
jeweils bezogen auf die Gesamtmasse des Dentalwerkstoffs.

10. Dentalwerkstoff nach Anspruch 9, der die folgende Zusammensetzung aufweist:
(a) 0,1 bis 20 Gew.-%, bevorzugt 0,2 bis 15 Gew.-% besonders bevorzugt 0,5 bis 10 Gew.-% mindestens eines Urethanallylsulfids der allgemeinen Formel I, II und/oder III, vorzugsweise I,
(b) 5 bis 50 Gew.-% , bevorzugt 10 bis 50 Gew.-% und besonders bevorzugt 20 bis 40 Gew.-% mindestens eines weiteren radikalisch polymerisierbaren Monomers,
(c) 0,01 bis 5,0 %, bevorzugt 0,1 bis 5,0 Gew.-% und besonders bevorzugt 0,2 bis 3,0 Gew.-% mindestens eines Initiators für die radikalische Polymerisation,
(d) 10 bis 70 Gew.-%, bevorzugt 20 bis 70 Gew.-% und besonders bevorzugt 30 bis 70 Gew.-% mindestens eines Füllstoffs und
(e) 0 bis 5 Gew.-%, bevorzugt 0,1 bis 5,0 Gew.-% und besonders bevorzugt 0,2 bis 4,0 Gew.-% eines oder mehrerer Additive,
jeweils bezogen auf die Gesamtmasse des Dentalwerkstoffs.

11. Dentalwerkstoff nach Anspruch 9, der die folgende Zusammensetzung aufweist:
(a) 0,1 bis 20 Gew.-%, bevorzugt 0,2 bis 15 Gew.-% besonders bevorzugt 0,5 bis 8 Gew.-% mindestens eines Urethanallylsulfids der allgemeinen Formel I, II und/oder III, vorzugsweise I,
(b) 10 bis 50 Gew.-%, bevorzugt 15 bis 40 Gew.-% und besonders bevorzugt 15 bis 30 Gew.-% mindestens eines weiteren radikalisch polymerisierbaren Monomers,
(c) 0,01 bis 5,0 Gew.-%, bevorzugt 0,1 bis 5,0 Gew.-% und besonders bevorzugt 0,1 bis 0,3 Gew.-% mindestens eines Initiators für die radikalische Polymerisation,
(d) 10 bis 85 Gew.-%, bevorzugt 20 bis 85 Gew.-% und besonders bevorzugt 30 bis 85 Gew.-% mindestens eines Füllstoffs und
(e) 0 bis 5 Gew.-%, bevorzugt 0,1 bis 5,0 Gew.-% und besonders bevorzugt 0,2 bis 3,0 Gew.-% eines oder mehrerer Additive,
jeweils bezogen auf die Gesamtmasse des Dentalwerkstoffs.

12. Dentalwerkstoff nach einem der Ansprüche 1 bis 11, der abgesehen von einer oder mehreren Verbindungen der Formeln I, II und III, keine flüchtigen Mercaptane, bevorzugt überhaupt keine Mercaptane und vorzugsweise auch keine anderen Schwefelverbindungen enthält.

13. Dentalwerkstoff nach einem der Ansprüche 1 bis 12 zur intraoralen Verwendung zur Restauration geschädigter Zähne, vorzugswese als Zement, Füllungskomposit oder Verblendmaterial.

14. Verwendung eines Dentalwerkstoffs nach einem der Ansprüche 1 bis 12 als Material zur extraoralen Herstellung oder Reparatur von Dentalrestaurationen, vorzugsweise von Inlays, Onlays, Kronen oder Brücken.

15. Extraorale Verwendung einer Verbindung der Formel I, II oder III, in der die Variablen wie in Anspruch 1 oder einem der Ansprüche 3 bis 4 definiert sind, zur Verringerung der Polymerisationsschrumpfungsspannung von Dentalwerkstoffen.

## Claims

1. Radically polymerizable dental material, which contains at least one compound of Formulae I to III: 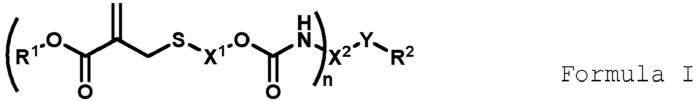 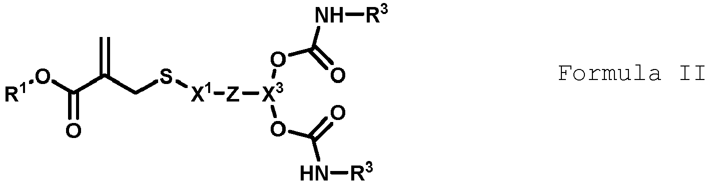 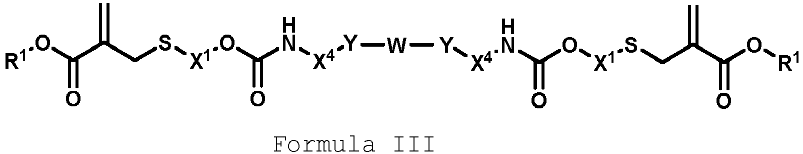 wherein the variables have the following meanings:
R¹ hydrogen, an aliphatic linear or branched C₁-C₁₅ alkyl radical, which can be interrupted by one or more, preferably 1 to 3, O or S and which can be substituted or unsubstituted, benzyl or phenyl;
R² hydrogen, an aliphatic linear or branched C₁-C₁₅ alkyl radical, which can be interrupted by one or more, preferably 1 to 3, O or S and which can be substituted or unsubstituted, benzyl, an aromatic C₆-C₁₈ radical, which can be substituted or unsubstituted, or a (meth)acryloyloxy group;
R³ an aliphatic linear or branched C₁-C₁₅ alkyl radical, which can be interrupted by one or more O and which can be substituted or unsubstituted, benzyl, or an aromatic C₆-C₁₈ radical, which can be substituted or unsubstituted;
X¹ a linear or branched aliphatic C₁-C₁₅ alkylene radical, which can be interrupted by one or more O or S, or a cycloaliphatic C₆-C₁₆ radical;
X² an (n+1)-valent organic C₁-C₂₀ radical, which can be interrupted by one or more O or S, a cycloaliphatic C₅-C₂₀ radical, an aromatic C₆-C₂₀ radical or an isocyanuric acid radical according to the following formula: 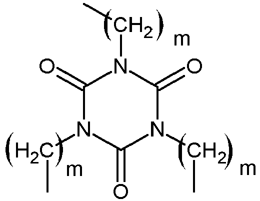
X³ a 3-valent linear or branched aliphatic C₃-C₂₀ radical;
X⁴ a 2-valent organic C₂-C₃₀ radical, which can be substituted by one or more methyl groups or is unsubstituted;
Y is absent or an ether (-O-), ester (-COO- or -OOC-), urethane (-NR⁴-CO-O- or -O-CO-NR⁴-) or amide group (-CONR⁴- or -NR⁴-CO-), wherein R⁴ in each case represents H or a C₁-C₃ alkyl radical;
Z is absent or an ether (-O-), ester (-COO- or -OOC-), or amide group (-CONR⁵- or -NR⁵-CO-), wherein R⁵ represents H or a C₁-C₃ alkyl radical;
W an oligomeric polyester, polyether or polythioether group;
m 1, 2, 3, 4, 5 or 6;
n 1, 2, 3 or 4,
wherein, if X² is an isocyanuric acid radical, then Y and R² are absent and n is 3.

2. Dental material according to claim 1, which contains at least one additional radically polymerizable monomer and preferably also at least one initiator for the radical polymerization.

3. Dental material according to claim 1 or 2, wherein the variables have the following meanings:
R¹ hydrogen, an aliphatic linear or branched C₁-C₁₀ alkyl radical, which can be interrupted by one or more, preferably 1 to 2, O and which can carry one or more, preferably 1 to 3, substituents, which are preferably selected from -CH₃, -C₂H₅ and/or polymerizable (meth)acryloyloxy groups, or is unsubstituted, benzyl or phenyl;
R² hydrogen, an aliphatic linear or branched C₁-C₁₀ alkyl radical, which can be interrupted by one or more, preferably 1 to 2, O and which can carry one or more, preferably 1 to 3, substituents, which are preferably selected from -CH₃, -C₂H₅ and/or polymerizable (meth)acryloyloxy groups, or is unsubstituted, benzyl, or an aromatic C₆-C₁₂ radical, which can carry one or more, preferably 1 to 3, substituents, which are preferably selected from -CH₃, -C₂H₅, and/or polymerizable (meth)acryloyloxy groups, or is unsubstituted, or a (meth)acryloyloxy group;
R³ an aliphatic linear or branched C₁-C₁₀ alkyl radical, which can carry one or more, preferably 1 to 3, substituents, which are preferably selected from -CH₃, -C₂H₅, halogen and/or polymerizable (meth)acryloyloxy groups, or is unsubstituted, benzyl, or an aromatic C₆-C₁₈ radical, which can carry one or more, preferably 1 to 3, substituents, which are preferably selected from -CH₃, -C₂H₅, polymerizable vinyl and/or (meth)acryloyloxy groups, or is unsubstituted;
X¹ a linear or branched aliphatic C₁-C₁₀ alkylene radical, which can be interrupted by one or more, preferably 1 to 2, O, or a cycloaliphatic C₆-C₁₂ radical;
X² an (n+1)-valent organic C₁-C₁₅ radical, preferably an aliphatic C₁-C₁₅ radical, which can be interrupted by one or more, preferably 1 to 2, O, a cycloaliphatic radical or an aromatic C₆-C₁₄ radical;
X³ a 3-valent linear or branched aliphatic C₃-C₁₀ radical;
X⁴ a 2-valent organic C₂-C₂₀ radical, preferably an aliphatic C₂-C₂₀ radical, a cycloaliphatic C₅-C₁₀ radical or a combination thereof, which can carry one or more, preferably 1 to 2, methyl groups as substituents, or is unsubstituted;
Y is absent or an ester or urethane group;
Z is absent or an ether or ester group;
W an oligomeric polyester or polyether group and
n 1, 2 or 3.

4. Dental material according to claim 3, wherein the variables have the following meanings:
R¹ an aliphatic linear or branched C₁-C₄ alkyl radical or benzyl (Ph-CH₂-), quite particularly preferably methyl or ethyl;
R² hydrogen or a (meth)acryloyloxy group;
R³ a linear C₁-C₃ alkyl radical, benzyl (Ph-CH₂-), phenyl (Ph-) or p-tolyl (H₃C-Ph-), wherein these radicals can be substituted in each case by a (meth)acryloyloxy group;
X¹ a linear C₁-C₃ alkylene radical;
X² an (n+1)-valent linear or branched aliphatic C₂-C₁₂ radical, which can be interrupted by 1 to 3 O, or an aromatic C₆-C₁₂ radical;
X³ a 3-valent linear or branched aliphatic C₃-C₄ radical; in particular -CH₂-CH(-)-CH₂-;
X⁴ a 2-valent aliphatic C₂-C₁₀ radical or a cycloaliphatic C₆ radical, which can be substituted with 1 to 3 methyl groups;
Y is absent or an ester or urethane group;
Z is absent;
W an oligomeric polyester or polyether group and
n 1, 2 or 3, in particular 1 or 2.

5. Dental material according to one of claims 1 to 4, wherein the substituents present in the case of the individual radicals are selected in each case from C₁-C₃ alkyl groups, preferably CH₃- and/or C₂H₅-, halogen, OH, OCH₃ or -O-COCH₃, polymerizable vinyl, (meth)acryloyloxy and/or (meth)acrylamide groups.

6. Dental material according to one of claims 1 to 5, which contains at least one multifunctional (meth)acrylate or a mixture of mono- and multifunctional (meth)acrylates.

7. Dental material according to one of claims 1 to 6, which contains at least one filler.

8. Dental material according to one of claims 1 to 7, which additionally contains at least one photoinitiator for the radical polymerization.

9. Dental material according to one of claims 1 to 8, which contains
(a) 0.1 to 30 wt.-%, preferably 0.2 to 25 wt.-% and particularly preferably 0.5 to 20 wt.-% of at least one compound of general formula I, II or III, preferably I,
(b) 10 to 99.1 wt.-%, preferably 10 to 95 wt.-% and particularly preferably 15 to 95 wt.-% of at least one further radically polymerizable monomer, preferably at least one multifunctional (meth)acrylate,
(c) 0.01 to 5.0 wt.-%, preferably 0.1 to 5.0 wt.-% and particularly preferably 0.1 to 3.0 wt.-% of at least one initiator for the radical polymerization,
(d) 0 to 90 wt.-%, preferably 0 to 85 wt.-% and particularly preferably 0 to 82 wt.-% of at least one filler, and
(e) 0 to 70 wt.-%, preferably 0.1 to 70 wt.-% and particularly preferably 0.1 to 60 wt.-% of one or more additives,
in each case relative to the total mass of the dental material.

10. Dental material according to claim 9, which has the following composition:
(a) 0.1 to 20 wt.-%, preferably 0.2 to 15 wt.-% and particularly preferably 0.5 to 10 wt.-% of at least one urethane allyl sulfide of general formula I, II and/or III, preferably I,
(b) 5 to 50 wt.-%, preferably 10 to 50 wt.-% and particularly preferably 20 to 40 wt.-% of at least one further radically polymerizable monomer,
(c) 0.01 to 5.0 wt.-%, preferably 0.1 to 5.0 wt.-% and particularly preferably 0.2 to 3.0 wt.-% of at least one initiator for the radical polymerization,
(d) 10 to 70 wt.-%, preferably 20 to 70 wt.-% and particularly preferably 30 to 70 wt.-% of at least one filler and
(e) 0 to 5 wt.-%, preferably 0.1 to 5.0 wt.-% and particularly preferably 0.2 to 4.0 wt.-% of one or more additives,
in each case relative to the total mass of the dental material.

11. Dental material according to claim 9, which has the following composition:
(a) 0.1 to 20 wt.-%, preferably 0.2 to 15 wt.-% and particularly preferably 0.5 to 8 wt.-% of at least one urethane allyl sulfide of general formula I, II and/or III, preferably I,
(b) 10 to 50 wt.-%, preferably 15 to 40 wt.-% and particularly preferably 15 to 30 wt.-% of at least one further radically polymerizable monomer,
(c) 0.01 to 5.0 wt.-%, preferably 0.1 to 5.0 wt.-% and particularly preferably 0.1 to 0.3 wt.-% of at least one initiator for the radical polymerization,
(d) 10 to 85 wt.-%, preferably 20 to 85 wt.-% and particularly preferably 30 to 85 wt.-% of at least one filler and
(e) 0 to 5 wt.-%, preferably 0.1 to 5.0 wt.-% and particularly preferably 0.2 to 3.0 wt.-% of one or more additives,
in each case relative to the total mass of the dental material.

12. Dental material according to one of claims 1 to 11, which, apart from one or more compounds of Formulae I, II and III, contains no volatile mercaptans, preferably no mercaptans at all and preferably also no other sulfur compounds.

13. Dental material according to one of claims 1 to 12 for intraoral use for the restoration of damaged teeth, preferably as cement, filling composite or veneering material.

14. Use of a dental material according to one of claims 1 to 12 as material for the extraoral production or repair of dental restorations, preferably of inlays, onlays, crowns or bridges.

15. Extraoral use of a compound of Formula I, II or III, in which the variables are as defined in claim 1 or one of claims 3 to 4, for reducing the polymerization shrinkage stress of dental materials.

## Revendications

1. Matériau de dentisterie, polymérisable par voie radicalaire, qui contient au moins un composé de l'une des formules I à III : 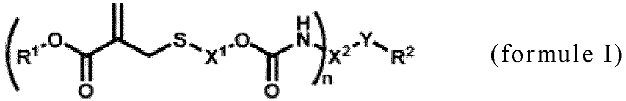 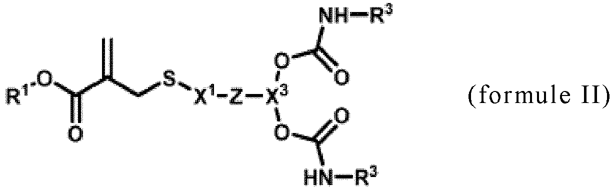 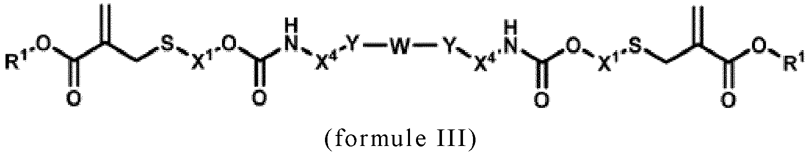 dans lesquelles les symboles ont les significations suivantes :
R¹ représente un atome d'hydrogène, un groupe alkyle aliphatique en C₁-C₁₅, linéaire ou ramifié, qui peut être interrompu par un ou plusieurs, de préférence 1 à 3, atome(s) d'oxygène ou de soufre et qui peut porter ou non un ou des substituant(s), un groupe benzyle ou un groupe phényle,
R² représente un atome d'hydrogène, un groupe alkyle aliphatique en C₁-C₁₅, linéaire ou ramifié, qui peut être interrompu par un ou plusieurs, de préférence 1 à 3, atome(s) d'oxygène ou de soufre et qui peut porter ou non un ou des substituant(s), un groupe benzyle, un groupe aromatique en C₆-C₁₈, qui peut porter ou non un ou des substituant(s), ou un groupe acryloyl-oxy ou méthacryloyl-oxy,
R³ représente un groupe alkyle aliphatique en C₁-C₁₅, linéaire ou ramifié, qui peut être interrompu par un ou plusieurs atome(s) d'oxygène et qui peut porter ou non un ou des substituant(s), un groupe benzyle, ou un groupe aromatique en C₆-C₁₈, qui peut porter ou non un ou des substituant(s),
X¹ représente un groupe alcanediyle aliphatique en C₁-C₁₅, linéaire ou ramifié, qui peut être interrompu par un ou plusieurs atome(s) d'oxygène ou de soufre, ou un groupe cycloaliphatique en C₆-C₁₆,
X² représente un groupe organique en C₁-C₂₀, de valence n+1, qui peut être interrompu par un ou plusieurs atome(s) d'oxygène ou de soufre, ou un groupe cycloaliphatique en C₅-C₂₀, un groupe aromatique en C₆-C₂₀, ou un groupe isocyanurate de formule suivante : 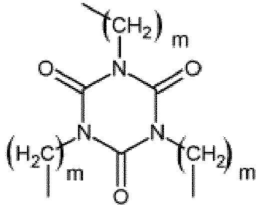
X³ représente un groupe aliphatique trivalent, en C₃-C₂₀, linéaire ou ramifié,
X⁴ représente un groupe organique divalent, en C₂-C₃₀, qui peut porter un ou plusieurs groupe(s) méthyle en tant que substituant(s) ou ne porter aucun substituant,
Y ne représente rien, ou représente un raccord de type éther -O-, ester -CO-O- ou -O-CO-, uréthane -NR⁴-CO-O- ou -O-CO-NR⁴-, ou amide -CO-NR⁴- ou -NR⁴-CO-, où R⁴ représente en chaque occurrence un atome d'hydrogène ou un groupe alkyle en C₁-C₃,
Z ne représente rien, ou représente un raccord de type éther -O-, ester -CO-O- ou -O-CO-, ou amide -CO-NR⁵- ou -NR⁵-CO-, où R⁵ représente un atome d'hydrogène ou un groupe alkyle en C₁-C₃,
W représente un groupe oligomère de type polyester, polyéther ou polythioéther,
m vaut 1, 2, 3, 4, 5 ou 6,
n vaut 1, 2, 3 ou 4 ;
étant entendu que, si X² représente un groupe isocyanurate, Y et R² ne représentent rien et l'indice n vaut 3.

2. Matériau de dentisterie, conforme à la revendication 1, qui contient au moins un monomère supplémentaire, polymérisable par voie radicalaire, ainsi que, de préférence, au moins un amorceur pour polymérisation radicalaire.

3. Matériau de dentisterie, conforme à la revendication 1 ou 2, dans lequel les symboles ont les significations suivantes :
R¹ représente un atome d'hydrogène, un groupe alkyle aliphatique en C₁-C₁₀, linéaire ou ramifié, qui peut être interrompu par un ou plusieurs, de préférence 1 à 2, atome(s) d'oxygène et qui peut porter un ou plusieurs, de préférence 1 à 3, substituant(s) choisi(s), de préférence, parmi les groupes méthyle et éthyle et/ou les groupes polymérisables acryloyl-oxy et méthacryloyl-oxy, ou bien ne porte aucun substituant, un groupe benzyle ou un groupe phényle,
R² représente un atome d'hydrogène, un groupe alkyle aliphatique en C₁-C₁₀, linéaire ou ramifié, qui peut être interrompu par un ou plusieurs, de préférence 1 à 2, atome(s) d'oxygène et qui peut porter un ou plusieurs, de préférence 1 à 3, substituant(s) choisi(s), de préférence, parmi les groupes méthyle et éthyle et/ou les groupes polymérisables acryloyl-oxy et méthacryloyl-oxy, ou bien ne porte aucun substituant, un groupe benzyle, un groupe aromatique en C₆-C₁₂, qui peut porter un ou plusieurs, de préférence 1 à 3, substituant(s) choisi(s), de préférence, parmi les groupes méthyle et éthyle et/ou les groupes polymérisables acryloyl-oxy et méthacryloyl-oxy, ou bien ne porte aucun substituant, ou un groupe acryloyl-oxy ou méthacryloyl-oxy,
R³ représente un groupe alkyle aliphatique en C₁-C₁₀, linéaire ou ramifié, qui peut porter un ou plusieurs, de préférence 1 à 3, substituant(s) choisi(s), de préférence, parmi les groupes méthyle et éthyle, les atomes d'halogène et/ou les groupes polymérisables acryloyl-oxy et méthacryloyl-oxy, ou bien ne porte aucun substituant, un groupe benzyle, ou un groupe aromatique en C₆-C₁₈, qui peut porter un ou plusieurs, de préférence 1 à 3, substituant(s) choisi(s), de préférence, parmi les groupes méthyle et éthyle et les groupes polymérisables vinyle et/ou acryloyl-oxy ou méthacryloyloxy, ou bien ne porte aucun substituant,
X¹ représente un groupe alcanediyle aliphatique en C₁-C₁₀, linéaire ou ramifié, qui peut être interrompu par un ou plusieurs, de préférence 1 à 2, atome(s) d'oxygène, ou un groupe cycloaliphatique en C₆-C₁₂,
X² représente un groupe organique en C₁-C₁₅, de valence n+1, de préférence un groupe aliphatique en C₁-C₁₅, qui peut être interrompu par un ou plusieurs, de préférence 1 à 2, atome(s) d'oxygène, un groupe cycloaliphatique, ou un groupe aromatique en C₆-C₁₄,
X³ représente un groupe aliphatique trivalent, en C₃-C₁₀, linéaire ou ramifié,
X⁴ représente un groupe organique divalent, en C₂-C₂₀, de préférence un groupe aliphatique en C₂-C₂₀, un groupe cycloaliphatique en C₅-C₁₀ ou une combinaison de tels groupes, qui peut porter un ou plusieurs, de préférence 1 à 2, groupe(s) méthyle en tant que substituant(s), ou bien ne porte aucun substituant,
Y ne représente rien, ou représente un raccord de type ester ou uréthane,
Z ne représente rien, ou représente un raccord de type éther ou ester,
W représente un groupe oligomère de type polyester ou polyéther,
n vaut 1, 2 ou 3.

4. Matériau de dentisterie, conforme à la revendication 3, dans lequel les symboles ont les significations suivantes :
R¹ représente un groupe alkyle aliphatique en C₁-C₄, linéaire ou ramifié, ou un groupe benzyle (Ph-CH₂-), mais, avec une préférence toute particulière, un groupe méthyle ou éthyle,
R² représente un atome d'hydrogène ou un groupe acryloyl-oxy ou méthacryloyl-oxy,
R³ représente un groupe alkyle linéaire en C₁-C₃, benzyle (Ph-CH₂-), phényle (Ph-) ou para-tolyle (H₃C-Ph-), étant entendu que ces groupes peuvent porter, en tant que substituant, un groupe acryloyl-oxy ou méthacryloyl-oxy,
X¹ représente un groupe alcanediyle linéaire en C₁-C₃,
X² représente un groupe aliphatique en C₂-C₁₂, linéaire ou ramifié, de valence n+1, qui peut être interrompu par 1 à 3 atome(s) d'oxygène, ou un groupe aromatique en C₆-C₁₂,
X³ représente un groupe aliphatique trivalent en C₃-C₄, linéaire ou ramifié, en particulier le groupe de formule -CH₂-CH(-)-CH₂-,
X⁴ représente un groupe aliphatique divalent en C₂-C₁₀ ou un groupe cycloaliphatique en C₆, qui peut porter 1 à 3 groupe(s) méthyle en tant que substituant(s),
Y ne représente rien, ou représente un raccord de type ester ou uréthane,
Z ne représente rien,
W représente un groupe oligomère de type polyester ou polyéther,
n vaut 1, 2 ou 3, en particulier 1 ou 2.

5. Matériau de dentisterie, conforme à l'une des revendications 1 à 4, dans lequel les substituants présents sur les divers groupes sont choisis pour chacun parmi les groupes alkyle en C₁-C₃, de préférence -CH₃ et/ou -C₂H₅, les atomes d'halogène, les groupes symbolisés par -OH, -OCH₃ ou -O-COCH₃, et les groupes polymérisables vinyle, acryloyl-oxy, méthacryloyl-oxy, acrylamido et/ou méthacrylamido.

6. Matériau de dentisterie, conforme à l'une des revendications 1 à 5, qui contient au moins un (méth)acrylate polyfonctionnel ou un mélange de (méth)acrylates monofonctionnels et polyfonctionnels.

7. Matériau de dentisterie, conforme à l'une des revendications 1 à 6, qui contient au moins une charge.

8. Matériau de dentisterie, conforme à l'une des revendications 1 à 7, qui contient en outre au moins un photoamorceur pour polymérisation radicalaire.

9. Matériau de dentisterie, conforme à l'une des revendications 1 à 8, qui contient
a) de 0,1 à 30 % en poids, de préférence de 0,2 à 25 % en poids, et mieux encore de 0,5 à 20 % en poids d'au moins un composé de formule générale I, II ou III, et de préférence I,
b) de 10 à 99,1 % en poids, de préférence de 10 à 95 % en poids, et mieux encore de 15 à 95 % en poids d'au moins un autre monomère polymérisable par voie radicalaire, de préférence d'au moins un acrylate ou méthacrylate polyfonctionnel,
c) de 0,01 à 5,0 % en poids, de préférence de 0,1 à 5,0 % en poids, et mieux encore de 0,1 à 3,0 % en poids d'au moins un amorceur pour polymérisation radicalaire,
d) de 0 à 90 % en poids, de préférence de 0 à 85 % en poids, et mieux encore de 0 à 82 % en poids d'au moins une charge,
e) et de 0 à 70 % en poids, de préférence de 0,1 à 70 % en poids, et mieux encore de 0,1 à 60 % en poids d'un ou de plusieurs adjuvant(s),
dans chaque cas par rapport au poids total du matériau de dentisterie.

10. Matériau de dentisterie, conforme à la revendication 9, qui présente la composition suivante :
a) de 0,1 à 20 % en poids, de préférence de 0,2 à 15 % en poids, et mieux encore de 0,5 à 10 % en poids d'au moins un uréthane-allyl-sulfure de formule générale I, II ou III, et/ou de préférence I,
b) de 5 à 50 % en poids, de préférence de 10 à 50 % en poids, et mieux encore de 20 à 40 % en poids d'au moins un autre monomère polymérisable par voie radicalaire,
c) de 0,01 à 5,0 % en poids, de préférence de 0,1 à 5,0 % en poids, et mieux encore de 0,2 à 3,0 % en poids d'au moins un amorceur pour polymérisation radicalaire,
d) de 10 à 70 % en poids, de préférence de 20 à 70 % en poids, et mieux encore de 30 à 70 % en poids d'au moins une charge,
e) et de 0 à 5 % en poids, de préférence de 0,1 à 5,0 % en poids, et mieux encore de 0,2 à 4,0 % en poids d'un ou de plusieurs adjuvant(s),
dans chaque cas par rapport au poids total du matériau de dentisterie.

11. Matériau de dentisterie, conforme à la revendication 9, qui présente la composition suivante :
a) de 0,1 à 20 % en poids, de préférence de 0,2 à 15 % en poids, et mieux encore de 0,5 à 8 % en poids d'au moins un uréthane-allyl-sulfure de formule générale I, II et/ou III, et de préférence I,
b) de 10 à 50 % en poids, de préférence de 15 à 40 % en poids, et mieux encore de 15 à 30 % en poids d'au moins un autre monomère polymérisable par voie radicalaire,
c) de 0,01 à 5,0 % en poids, de préférence de 0,1 à 5,0 % en poids, et mieux encore de 0,1 à 0,3 % en poids d'au moins un amorceur pour polymérisation radicalaire,
d) de 10 à 85 % en poids, de préférence de 20 à 85 % en poids, et mieux encore de 30 à 85 % en poids d'au moins une charge,
e) et de 0 à 5 % en poids, de préférence de 0,1 à 5,0 % en poids, et mieux encore de 0,2 à 3,0 % en poids d'un ou de plusieurs adjuvant(s),
dans chaque cas par rapport au poids total du matériau de dentisterie.

12. Matériau de dentisterie, conforme à l'une des revendications 1 à 11, lequel, mis à part le ou les composé(s) de formule I, II ou III, ne contient pas de mercaptans volatils, de préférence, ne contient pas du tout de mercaptans, et mieux encore, ne contient même pas d'autres composés soufrés.

13. Matériau de dentisterie, conforme à l'une des revendications 1 à 12, pour utilisation intrabuccale en vue de la restauration de dents abimées, de préférence en tant que ciment, matériau composite d'obturation ou matériau de placage.

14. Utilisation d'un matériau de dentisterie, conforme à l'une des revendications 1 à 12, en tant que matériau pour fabrication ou réparation extrabuccale de restaurations dentaires, et de préférence d'incrustations inlay ou onlay, de couronnes ou de bridges.

15. Utilisation extrabuccale d'un composé de formule I, II ou III, dans lesquelles formules les symboles ont les significations indiquées dans la revendication 1 ou dans l'une des revendications 3 et 4, pour réduire le retrait subi par des matériaux de dentisterie lors de leur polymérisation.
